(19) Europäisches Patentamt
European Patent Office
Office européen des brevets

(11) EP 0 390 135 B1

(12) # EUROPEAN PATENT SPECIFICATION

(45) Date of publication and mention of the grant of the patent: **05.06.1996 Bulletin 1996/23**

(51) Int. Cl.⁶: **C07D 471/06**, C07D 209/30, C07B 57/00, A61K 31/47 // (C07D471/06, 221:00, 209:00)

(21) Application number: **90106003.8**

(22) Date of filing: **29.03.1990**

(54) **Pyrroloquinoline derivatives, antimicrobial agents using the same and process for preparing the same**

Pyrrolochinolin-Derivate, ihre Verwendung in antimikrobiellen Mitteln und Verfahren zu ihrer Herstellung

Dérivés de pyrroloquinoline, leur utilisation comme agents antimicrobiens et procédé pour leur préparation

(84) Designated Contracting States:
**CH DE DK ES FR GB IT LI NL SE**

(30) Priority: **29.03.1989 JP 78786/89**

(43) Date of publication of application:
**03.10.1990 Bulletin 1990/40**

(73) Proprietor: **OTSUKA PHARMACEUTICAL CO., LTD.**
**Chiyoda-ku Tokyo 101 (JP)**

(72) Inventors:
- **Fujii, Setsuro deceased (JP)**
- **Ishikawa, Hiroshi Shiga, 520-02 (JP)**
- **Tsubouchi, Hidetsugu Shiga, 502 (JP)**
- **Jitsukawa, Koichiro Hyogo, 659 (JP)**

(74) Representative: **Brauns, Hans-Adolf, Dr. rer. nat. et al**
**Hoffmann, Eitle & Partner**
**Patentanwälte**
**Postfach 81 04 20**
**81904 München (DE)**

(56) References cited:
**EP-A- 0 171 616**
**WO-A-87/03587**
**DE-A- 2 914 258**
**GB-A- 2 086 905**

- **PATENT ABSTRACTS OF JAPAN, vol. 8, no. 184 (C-239)(1621)**
- **PATENT ABSTRACTS OF JAPAN, vol. 7, no. 19 (C-147)(1164)**
- **PATENT ABSTRACTS OF JAPAN, vol. 7, no. 83 (C-160)(1228)**
- **PATENT ABSTRACTS OF JAPAN, vol. 10, no. 182 (C-356)(2238)**
- **PATENT ABSTRACTS OF JAPAN, vol. 8, no. 241 (C-250)(1678)**

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 390 135 B1

Printed by Rank Xerox (UK) Business Services
2.11.9/3.4

## Description

This invention relates to pyrroloquinoline derivatives, antimicrobial agents using the same and process for preparing the same.

Various pyrroloquinoline derivatives are known which have antimicrobial activity. JP-A-59078189 describes pyrrolo[3,2,1-ij]quinoline-5-carboxylic acid derivatives having antimicrobial activity. Furthermore, there are disclosed in GB-2020279 a piperazinylbenzoheterocyclic compound represented by the formula

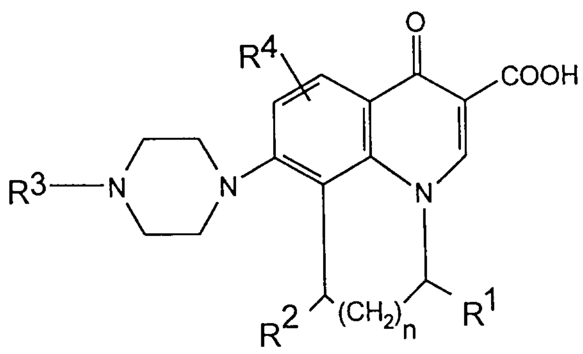

However, compounds which are more excellent in their antimicrobial activity are desired.

The main object of this invention is to provide pyrroloquinoline derivatives or salts thereof having excellent antimicrobial activity and to provide antimicrobial agents using the same and process for preparing the same.

Namely, this invention provides pyrroloquinoline derivatives or thereof of the following general formula (I) which are optically active due to the asymmetric carbon atom at the 2-position.

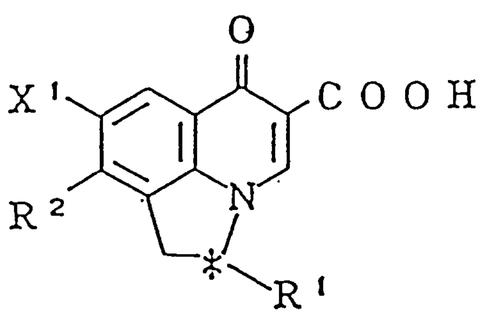

(I)

wherein $R^1$ is a $C_1$-$C_6$ alkyl group; $R^2$ is a group:

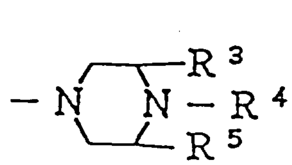

wherein $R^3$, $R^4$ and $R^5$ are the same or different, a hydrogen atom or a $C_1$-$C_6$ alkyl group, provided that at least one of the groups of $R^3$, $R^4$ and $R^5$ is the $C_1$-$C_6$ alkyl group, $X^1$ is a halogen atom and * denotes an asymmetric atom, which has optical activity due to the asymmetric carbon atom at the 2-position wherein the 2-position asymmetric carbon atom of pyrroloquinoline ring has the same configuration as the 2-position asymmetric carbon atom of one of the two diastereoisomers of (4,5-difluoro-2-methyl-1-[(S)-N-p-toluenesulfonylprolyl]-2,3-dihydroindole which show an $[a]_D^{20}$ = - 10.38° (c = 1.06, chloroform)), or salt thereof.

The subclaims are directed to preferred embodiments.

The compounds of this invention represented by the general formula (I) described above which are optically active due to the asymmetric carbon atom at the 2-position are so excellent in their antimicrobial activity that they are useful as antimicrobial compounds. In addition the compounds (I) of this invention are characterized by low toxicity, long duration of effect, and good absorption when orally administered. The compounds (I) of this invention are particularly suitable for use in the form of injections because of their high solubility in water. And the compounds (I) has low centric action.

The optically active compounds of this invention (I) are classified into two types describe below.

The compounds (a) and (b) are contained in the compounds of general formula (II) which are optically active.

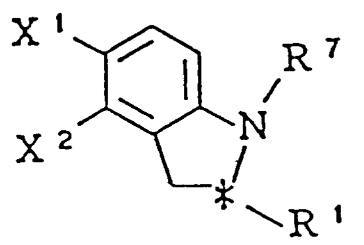

(II)

wherein $R^1$, $X^1$, and * have the same meanings as defined above; $X^2$ is a halogen atom; $R^7$ is a hydrogen atom or the group

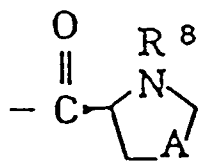

in which $R^8$ is a protective group selected from $C_1$-$C_6$ alkyl-substituted phenylsulfonyl groups and $C_{1-6}$ alkyl-substituted phenylcarbonyl groups; and A is a $C_1$-$C_6$ alkylene group.

The optically active compounds represented by the general formula (II) are useful as the intermediates for the synthesis of the compounds (I) of this invention.

In another aspect, this invention provides the pyrroloquinoline derivatives represented by the general formula (III).

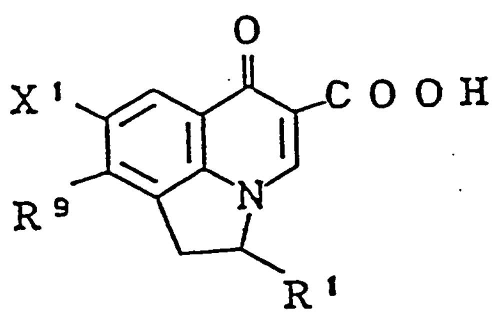

(III)

wherein $R^1$ is a $C_1$-$C_6$ alkyl group; $R^9$ is a group:

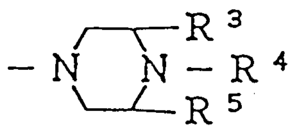

wherein $R^3$ is a $C_1$-$C_6$ alkyl group, and $R^4$ and $R^5$ are hydrogen atoms. $X^1$ is a halogen atom or a salt thereof which is a racemic modification.

The pyrroloquinoline derivatives of the general formula (III) are excellent in their antimicrobial activity, and therefore useful as antimicrobial agents. The compounds (III) of this invention are characterized by low toxicity, long duration of effect, and good absorption when orally administered, hurhter good solubility in water and low centric action.

In another aspect, this invention provides the pyrroloquinoline derivatives represented by the following general formula (IV).

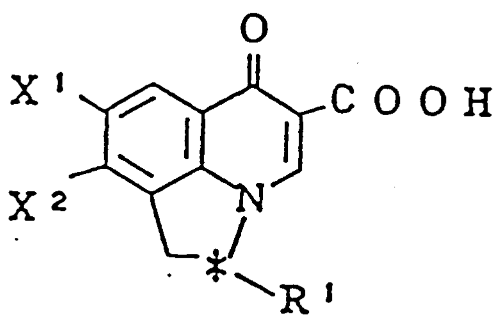

(IV)

wherein $R^1$ is a $C_1$-$C_6$ alkyl group; $X^1$ and $X^2$ is halogen atoms; and * denotes the asymmetric carbon atom.

The compounds of this invention are usefull as the intermediate for the salts which have good solubility in water. Hydrochloride sulfate and methansulfonate are particularly desiable. Among them hydrocholoride is especially desiable.

DETAILED DESCRIPTION OF THE INVENTION

The groups given in this specification are respectively described in more detail in the following.

The halogen atoms include fluorine, chlorine, bromine, and iodine atoms, among which fluorine and chlorine atoms are particularly desirable.

Examples of the $C_1$-$C_6$ alkyl groups include straight chain or branched alkyl groups, such as methyl, ethyl, propyl, isopropyl, butyl, tert-butyl, pentyl, hexyl,. Among these, methyl and ethyl are desirable, and methyl is particularly desirable.

Examples of the protective groups represented by $R^8$ include $C_1$-$C_6$ alkyl-substituted phenylsulfonyl groups, $C_1$-$C_6$ alkyl-substituted phenylcarbonyl groups The $C_1$-$C_6$ alkyl-substituted phenylsulfonyl groups are exemplified by phenylsulfonyl groups substituted by straight chain or branched alkyl groups, such as p-toluenesulfonyl group. The $C_1$-$C_6$ alkyl-substituted phenylcarbonyl groups are exemplified by phenylcarbonyl groups substituted by straight chain or branched alkyl groups, such as 4-methylbenzoyl group.

The compounds of this invention are prepared for example according to the following reaction scheme.

[Reaction Scheme]

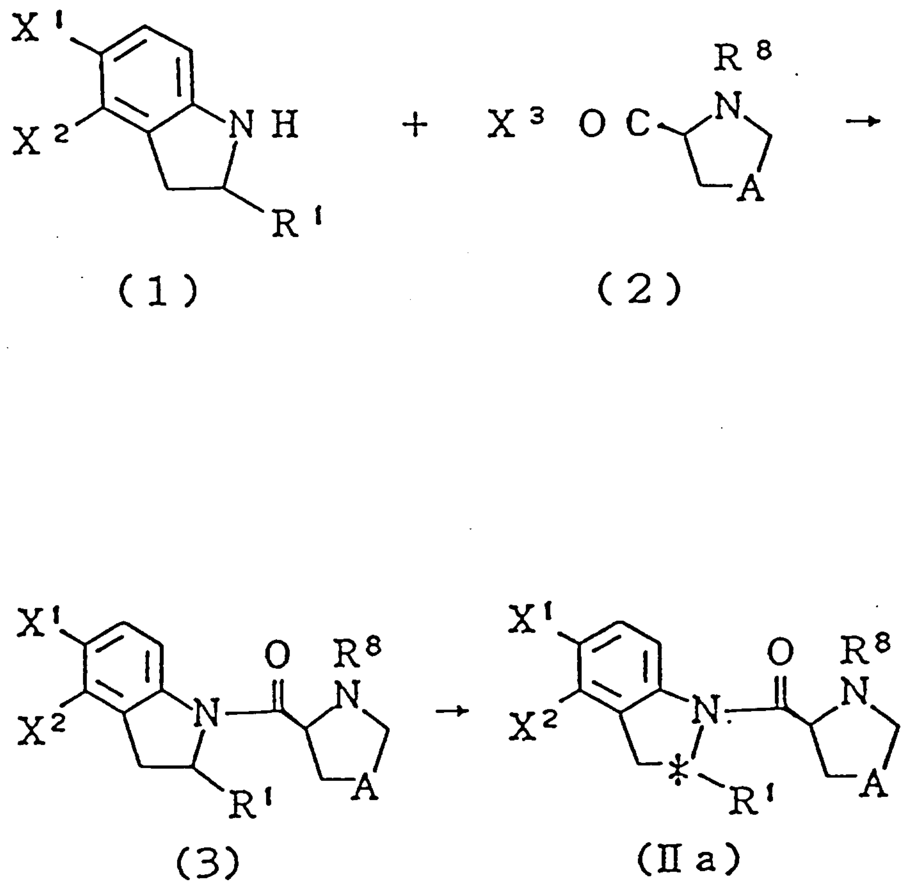

(1) (2)

(3) (IIa)

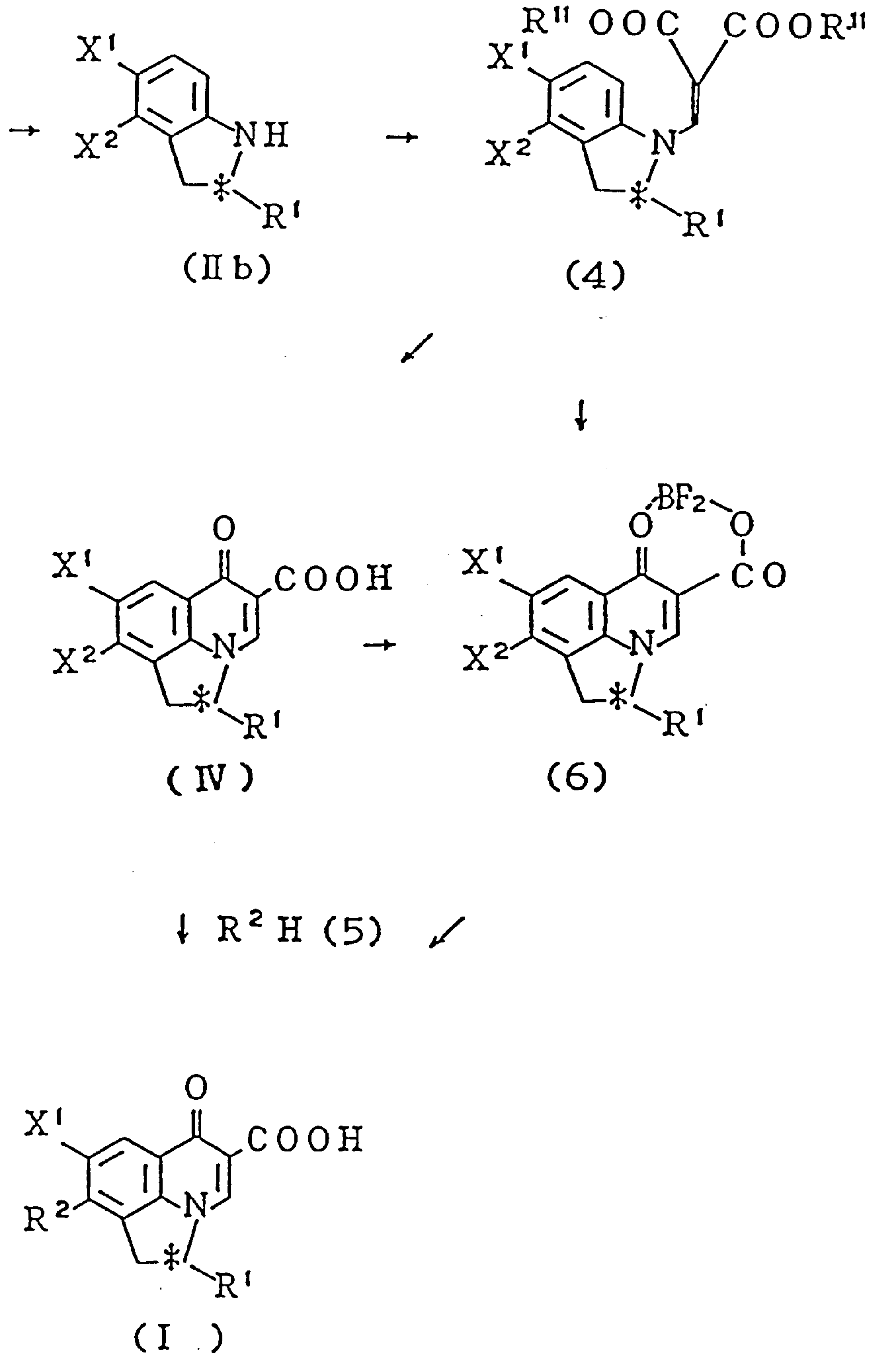


(IIb) (4) (IV) (6) (I)

wherein $R^1$, $R^2$, $R^3$, $R^4$, $R^5$, $R^6$, $R^7$, $R^8$, $R^9$, $R^{10}$, $X^1$, $X^2$, A and * have the same meanings as defined above; $X^3$ is a hydroxyl group, or a halogen atom, or a reactive derivative residue of a carboxyl group; $R^{11}$ is a $C_1$-$C_6$ alkyl group.

The reactive derivative residues of carboxyl group represented by $X^3$ in the general formula shown above include active ester residues, acid anhydride residues and the like.

The condensation between the compound (1) and a cyclic amino acid or its reactive derivative (2) is carried out by any of the usual methods for amide bond-forming reaction (for example, the acid halide method, the active ester method, the acid anhydride method, the DCC method, etc.).

For example, according to the acid halide method, the compound (3) can be produced by reacting the racemic compound (1) with an acyl halide (2) wherein $X^3$ is a halogen atom in the presence of a deoxidizer in a suitable solvent. The deoxidizers that can be used in this invention include sodium hydrogencarbonate, sodium carbonate, potassium carbonate, pyridine, triethylamine, etc. The solvents that can be used in this invention include benzene, halogenated hydrocarbons such as chloroform, methylene chloride and carbon tetrachloride, and ethers such as dioxane and tetrahydrofuran. The acyl halide (2) and the compound (1) are present at least in a molar ratio of about 1:1, preferably 1:1 to 3:1. The reaction is conducted usually at -30°C to about 100°C, preferably room temperature to about 80°C, and completes in 20 minutes to 20 hours.

The optically active compound (IIa) can be separated from the compound (3) by fractional crystallization or chromatography on silica gel, or by combination thereof.

The configuration at the asymmetric carbon atom of the 2-position in the optically active compound (IIa) is the same as that in the Compound a of Example 1 described below.

The compound (IIb) can be obtained by hydrolysis of the optically active compound (IIa) separated. The hydrolysis is conducted in a solvent in the presence of a catalyst conventionally used for hydrolysis. The catalysts for hydrolysis include basic compounds such as sodium hydroxide, potassium hydroxide, barium hydroxide, etc., mineral acids such as sulfuric acid, hydrochloric acid, nitric acid, etc., and organic acids such as acetic acid, aromatic sulfonic acid, etc. The solvents include water, lower alcohols such as methanol, ethanol, isopropanol, etc., ketones such as acetone, methylethylketone, etc., ethers such as dioxane, diethylene glycol, etc., acetic acid, and so on. This reaction is carried out usually at room temperature to about 200°C, preferably at about 50° to about 150°C.

The compound (4) can be obtained by reacting the compound (IIb) with dialkylethoxymethylene malonate. The said reaction may be carried out either in the absence of a solvent or in the presence of a solvent, preferably in the absence of a solvent. The solvents that can be used in the reaction include aromatic hydrocarbons such as benzene, toluene, xylene, etc., acetonitrile, dimethylformamide, dimethyl sulfoxide, hexamethylphosphoric triamide, and so on. Dialkylethoxymethylene malonate and the compound (IIb) may be used usually in a molar ratio of 1:1 or more; in the reaction in the absence of a solvent the ratio is preferably 1:1 and in the reaction in a solvent the ratio is preferably about 1.1:1 to about 1.5:1. The reaction is carried out usually at room temperature to about 150°C, preferably at about 100 - 130°C, and completes usually in about 0.5 - 6 hours.

The cyclization of the compound (4) can be conducted according to the known various cyclization procedures. In the concrete, as examples of the cyclization procedure, there may be mentioned cyclization by heating, and cyclization using an acidic substance such as phosphorus oxide chloride, phosphorus pentachloride, phosphorus trichloride, thionyl chloride, concentrated sulfuric acid, polyphosphoric acid, etc. The cyclization by heating is carried out in a suitable solvent usually at about 100°C to about 250°C, preferably about 150°C to about 200°C. The solvents that can be used include hydrocarbons of high boiling point and ethers of high boiling point, such as tetralin, diphenyl ether, diethylene glycol dimethyl ether, etc. In the cyclization using an acidic substance, equimolar amount to a large excess of the acidic substance is used for the amount of the compound (4), preferably 10 to 20 times moles of the acidic substance is used for a mole of the compound (4); the reaction is carried out usually at about 100°C to about 150° and completes in about 0.5 to about 6 hours. The cyclization produces an ester derivative of the compound of the general formula (IV).

The compound (IV) can be produced by hydrolysis of the above-mentioned ester derivative described above in a solvent in the presence of a conventional catalyst for hydrolysis. As the catalysts and the solvents, those used for the hydrolysis of the optically active compound (IIa) described above can be used. The said reaction is usually carried out at room temperature to about 200°C, preferably about 50°C to about 150°C.

The reaction between the compound (IV) and the compound (5) represented by the general formula $R^2H$ (wherein $R^2$ has the same meaning as defined above) is carried out in an inert solvent. As examples of the said solvent there may be mentioned water, alcohols such as methanol, ethanol, isopropanol, butanol, amyl alcohol, isoamyl alcohol, etc., aromatic hydrocarbons such as benzene, toluene, xylene, etc., ethers such as tetrahydrofuran, dioxane, diglyme, etc., dimethyl sulfoxide, dimethylformamide, hexamethylphosphoric triamide, and so on. Among these solvents, dimethyl sulfoxide, dimethylformamide, and hexamethylphosphoric triamide are particularly desirable. The molar ratio of the compound (IV) and the compound (5) is not specified and can be employed suitably from a wide range; for one mole of the compound (IV), usually one mole or more, preferably one mole to about 5 moles of the compound (5) is used. The said reaction is carried out usually at about 1 atm to about 20 atm, preferably at about 1 atm to about 10 atm, and at about 50°C to about 200°C, preferably at about 80°C to about 150°C, and completes usually in about 1 hour to about 10 hours. This reaction may be carried out in the presence of a deoxidizer. As examples of the deoxidizer, there may be mentioned inorganic carbonates such as sodium carbonate, potassium carbonate, potassium hydrogencarbonate, sodium hydrogencarbonate, tertiary amines such as pyridine, quinoline, triethylamine, etc., and so on.

The compound (I) can also be produced by reacting the chelate compound (6) obtained either from the compound (4) or from the compound (IV), with the compound (5).

The reaction to obtain the chelate compound (6) is carried out by reacting the compound (4) or the compound (IV) with boron trifluoride or a complex thereof in an inert solvent. Complexes of boron trifluoride that can be used include ether complex, acetic acid complex, ketone complexes, etc. Boron trifluoride or a complex thereof and the compound (4) or the compound (IV) are present usually in a molar ratio of 1:1 or more, preferably 1:1 to about 2:1. As examples of the inert solvent, there may be mentioned aromatic hydrocarbons such as benzene, toluene, xylene, etc., and ethers such as tetrahydrofuran, diethyl ether, etc. The reaction is carried out usually at about 100°C to about 200°C, preferably at about 100°C to about 150°C, and completes usually in about 3 hours to about 10 hours.

The reaction between the chelate compound (6) and the compound (5) is carried out in a similar manner as the reaction between the compound (IV) and the compound (5) described above. The said reaction is carried out usually at about 50°C to about 150°C, preferably at about 50°C to about 100°C, and completes usually in about 5 hours to about 20 hours.

Among the compounds represented by the formula (I) those wherein $R^2$ is

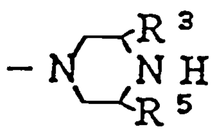

can be transformed into the compounds wherein $R^2$ is

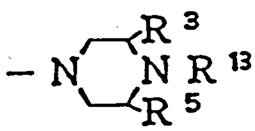

(in which $R^{13}$ means a $C_1$-$C_6$ alkyl group) by a known method for alkylation. The methods for alkylation include (i) a method wherein a compound represented by the general formula $R^{13}X^4$ (in which $R^{13}$ is a $C_1$-$C_6$ alkyl group) is allowed to react in the presence of a deoxidizer, and (ii) a method wherein a lower alkanol is allowed to react in the presence of a reducing agent. For example according to the method (ii), the reaction is carried out in the presence of a reducing agent in the absence of a solvent or in a suitable solvent. As examples of the reducing agent, there may be mentioned formic acid, sodium formate-formic acid, sodium borohydroxide, lithium aluminum hydroxide, etc. As examples of the solvent there may be mentioned aromatic hydrocarbons such as benzene, toluene, etc., halogenated hydrocarbons such as chloroform methylene chloride, etc., ethers such as tetrahydrofuran, etc., and so on. This reaction is carried out usually at about 50°C to about 200°C, preferably at about 50°C to about 150°C, and completes usually in about 3 hours to about 10 hours.

Among the optically active compounds according to the present invention, those that can form salts can also be produced from the corresponding racemic compounds.

For example, the optically active compound (IIb) can be obtained from the racemic compound (1). Namely, the salt of the compound (1) is formed by reacting the compound (1) with an optically active compound, and subjected to fractional crystallization, followed by desalting of the resultant salt of the optically active compound (IIb), to give the compound (IIb).

As the optically active compounds used for salt formation of the compound (1), a variety of known compounds may be used as far as they are optically active compounds that can form salts with the compound (1), including optically active acids such as (+)-and (-)-tartaric acid, (-)-malic acid, (-)-mandelic acid, and D-and L-camphor-10-sulfonic acid. Among these, D- and L-camphor-10-sulfonic acid is particularly desirable. As the solvent used for the salt-formation, any of those used for usual optical resolution can be used, including water, alcohols such as methanol ethanol, isopropanol, etc., aromatic hydrocarbons such as benzene, toluene, xylene, etc., halogenated hydrocarbons such as dichloromethane, chloroform, carbon tetrachloride, etc., ethers such as tetrahydrofuran, dioxane, diglyme, etc., aliphatic hydrocarbons such as n-hexane, n-heptane, cyclohexane, etc., polar solvents such as dimethyl sulfoxide, dimethylformaide, hexamethylphosphoric triamide, etc., and the mixtures thereof. The optically active compound and the compound (1) are present usually in a molar ratio of about 0.3:1 to about 3:1, preferably in a molar ratio of about 0.5:1 to about 1:1. This reaction proceeds usually at about 0°C to about 100°C, preferably at room temperature to about 50°C.

As the method for fractional crystallization of the salt of the compound (1) formed as described above, any of the known methods can be employed.

Desalting of the salt of the compound (IIb) obtained by fractional crystallization is carried out in a suitable solvent in the presence of a basic compound. As the solvent that can be used here any of those used in the salt-formation described above may be used. As examples of the basic compound there may be mentioned inorganic salts such as

sodium hydroxide, sodium carbonate, potassium hydroxide, potassium carbonate, potassium hydrogencarbonate, sodium hydrogencarbonate, etc. Desirably an excess of the basic compound is used.

All of the optically active compounds according to the present invention each of which has an asymmetric carbon at the 2-position have the same configuration at the 2-position as that in the compound (a) of Example 1 described below.

The compounds according to the present invention can be easily converted to acid adducts by permitting a pharmaceutically acceptable acid to act thereon. The acid is exemplified by inorganic acids such as hydrochloric acid, sulfuric acid, phosphoric acid, hydrobromic acid, etc., and organic acids such as oxalic acid, maleic acid, fumaric acid, malic acid, tataric acid, citric acid, benzoic acid, etc.

In addition, the compounds according to the present invention can be easily converted to salts by permitting a pharmaceutically acceptable basic compound to act thereon. The basic compound is exemplified by sodium hydroxide, potassium hydroxide, calcium hydroxide, sodium carbonate, potassium hydrogencarbonate, etc.

The thus-produced compound of this invention can be isolated from the reaction system and purified without difficulty by conventional means of separation such as distillation, recrystallization, column chromatography, preparative thin layer chromatography, and solvent extraction.

The compounds and the salts thereof represented by the general formula (I), (III) and (IV) according to the present invention are used as antimicrobial agents in the form of pharmaceutical preparations obtained by combining the compounds or the salts thereof with suitable pharmaceutically acceptable carriers. Any of the conventional carriers can be employed, including excipients, binders, lubricants, coloring agents, disintegrants, etc.

Various dosage forms of the antimicrobial agents can be selected according to the purpose of the therapy. In the concrete, the dosage forms include tablets, pills, powders, liquid preparations, suspensions, emulsions, granules, capsules, suppositories, and injectable preparations (solutions, suspensions, etc.), ointments, etc.

The compounds of this invention are used in the form of ordinary pharmaceutical preparations. The preparation is formulated by using diluents or excipients such as fillers, extenders, binders, wetting agents, disintegrants, surfactants, lubricants, etc. Various dosage forms of these pharmaceutical preparations can be selected according to the purpose of the therapy, and typical forms include tablets, pills, powders, suspensions, emulsions, granules, capsules, suppositories, injections (solutions, suspensions, etc.), ointments, and so on. In molding a pharmaceutical composition into a tablet form, examples of the carriers include excipients such as lactose, white sugar, sodium chloride, glucose, urea, starch, calcium carbonate, kaolin, crystalline cellulose, and silicic acid, binders such as water, ethanol, propanol, simple syrup, glucose solution, starch solution, gelatin solution, carboxymethylcellulose, shellac, methylcellulose, potassium phosphate and polyvinyl pyrrolidone, disintegrants such as dried starch, sodium alginate, agar powder, laminaria powder, sodium hydrogencarbonate, calcium carbonate, polyoxyethylenesorbitan fatty acid esters, sodium laurylsulfate, stearic acid monoglyceride, starch, and lactose, disintegration inhibitors such as white sugar, stearin, cacao butter and hydrogenated oils, absorption promoters such as quaternary ammonium bases and sodium laurylsulfate, humectants such as glycerol and starch, adsorbents such as starch, lactose, kaolin, bentonite and colloidal silicic acid, and lubricants such as purified talc, stearic acid salts, boric acid powder, polyethylene glycol. The tablets, if desired, can be coated, and made into sugar-coated tablets, gelatin-coated tablets, enteric-coated tablets, film-coated tablets, or tablets comprising two or more layers. In molding the pharmaceutical composition into pills, examples of the carriers include excipients such as glucose, lactose, starch, cacao butter, hardened vegetable oils, kaolin and talc, binders such as gum arabic powder, tragacanth powder, gelatin, and ethanol, and disintegrants such as laminaria and agar. In molding the pharmaceutical composition into a suppository form, examples of the carriers include polyethylene glycol, cacao butter, higher alcohols, esters of higher alcohols, gelatin, and semisynthetic glycerides. Capsules are prepared by filling the compounds of this invention mixed with various carriers described above as examples into hard gelatin capsules and soft capsules, according to the conventional procedure. When the pharmaceutical composition is formulated into an injectable preparation, the resultant solution, emulsion and suspension are sterilized, and are desirably isotonic with respect to the blood. In formulating, emulsion or suspension, examples of the diluents include water, aqueous solution of lactic acid, ethyl alcohol, propylene glycol, ethoxylated isostearyl alcohol, polyoxidized isostearyl alcohol, and polyoxyethylenesorbitan fatty acid esters. Sodium chloride, glucose or glycerol may be incorporated into a pharmaceutical composition, in an amount sufficient to prepare isotonic solutions. The pharmaceutical composition may further contain ordinary dissolving aids, buffers, painalleviating agents, and optionally coloring agents, preservatives, perfumes, flavors, sweeteners and other drugs. In molding a pharmaceutical composition into a paste form, a cream form, and a gel form, examples of the diluents include white petrolatum, paraffin, glycerin, cellulose derivatives, polyethylene glycol, silicones, and bentonite.

The amount of the compound of the present invention to be incorporated into a pharmaceutical composition is not particularly limited, and can be selected appropriately from a wide range, being usually about 1 to about 70% by weight.

The administration method of the pharmaceutical composition according to the invention is not particularly limited and can be adequately selected according to the form of the preparation, age, sex and other conditions of the patient, severity of the disease, etc. For example, the tablets, pills, liquid preparations, suspensions, emulsions, granules, and capsules are orally administered. The injectable preparations are intravenously administered either alone or together with ordinary auxiliary agents such as glucose and amino acids. Furthermore, as required, the injectable preparations

can be singly administered intramuscularly, intracutaneously, subcutaneously, or intraperitoneally. The suppository is administered intraectally. The ointment is applied on the skin.

The dosage of the pharmaceutical composition is suitably selected according to the purpose of use, age, sex, and other conditions of the patient, and severity of the disease, etc. Usually, the pharmaceutical composition containing a preferred dosage of the compound of this invention as the effective ingredient of about 10 mg to about 5 g/body may be administered by dividing into 3 to 4.

BRIEF DESCRIPTION OF THE DRAWINGS

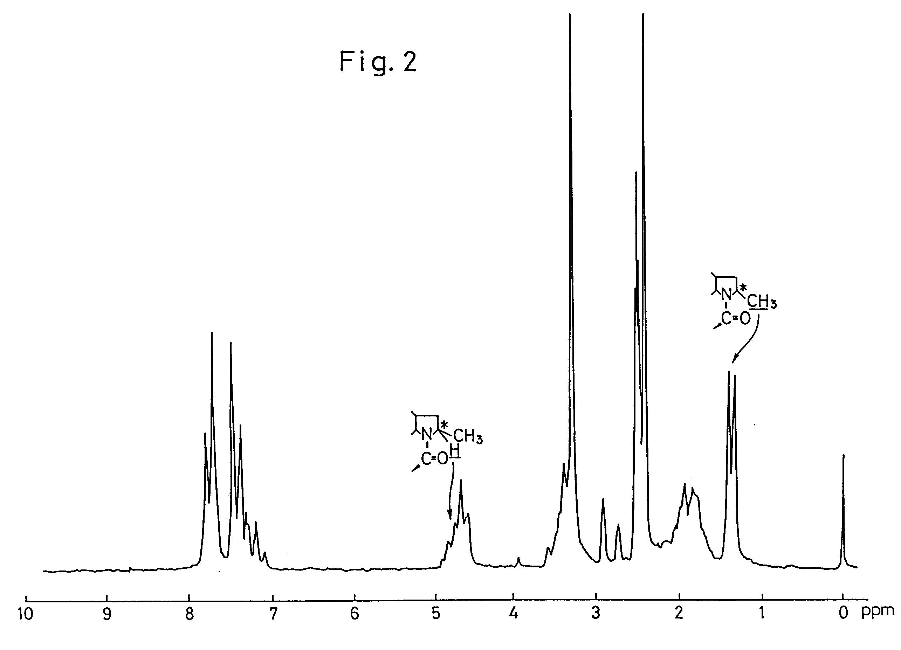

Fig.1 is the $^1$H-NMR spectrum of the Compound a; and Fig.2 is the $^1$H-NMR spectrum of the Compound b.

EXAMPLES

Hereinafter, Examples, Reference Example, Pharmacological Activity Test Example, and Pharmaceutical Examples are described.

Example 1

(±)-4,5-difluoro-2-methyl-2,3-dihydroindole, 47.4 g, was dissolved in 470 mℓ of methylene chloride, to which was added 45 mℓ of pyridine under ice-cooling. To the resultant mixture was added dropwise under ice-cooling a solution of the acid chloride prepared from 98.8 g of (S)-N-p-toluenesulfonyl proline and 80 mℓ of thionyl chloride in 300 mℓ of methylene chloride. After the dropwise addition, the reaction was conducted for 1.5 hours at room temperature. The reaction mixture was washed with 750 mℓ of 10% hydrochloric acid, with a saturated aqueous solution of sodium hydrogencarbonate, and with a saturated aqueous solution of sodium chloride, in this order, and dried over anhydrous magnesium sulfate. Methylene chloride was evaporated off, the residue was washed with ether, with benzene and with ethanol, in this order. All of the washings were combined and concentrated, and the residue was subjected to column chromatography on 3 kg of silica gel with elution with benzene-ethyl acetate (30:1 - 15:1), to give a main product and a by-product. The main product was recrystallized from isopropanol-benzene, to give 51.8 g of (-)-4,5-difluoro-2-methyl-1-[(S)-N-p-toluenesulfonylprolyl]-2,3-dihydroindole (Compound a) having the following physical characteristics.

white edged crystals, mp: 168 - 170°C

$[\alpha]_D^{20}$ = -10.38° (c=1.06, chloroform)

| Elemental analysis for $C_{21}H_{22}F_2N_2O_3S$ | | | |
|---|---|---|---|
| Calcd.: | C:59.90, | H:5.27, | N:6.66 |
| Found : | C:59.73, | H:5.24, | N:6.74 |

The NMR spectrum of the Compound a is shown in Fig.1. The typical peaks are shown below.

$^1$H-NMR (DMSO-$d_6$/TMS) δ:

1.26 (3H, d, J=6.15Hz), 1.63 - 2.2 (4H, m), 2.36 (3H, s), 2.8 (1H, d, J=13.5Hz), 3.2 - 3.7 (3H, m), 4.5 - 4.7 (1H, m), 4.8 - 5.2 (1H, m), 7.3 (2H, d, J=8.35Hz), 7.7 (2H, d, J=8.35Hz 7.07 - 7.5 (1H, m), 7.6 - 7.9 (1H, m)

When the Compound a having the physical characteristics shown above is used as the starting substance, the compounds of this invention in Examples 3, 5, 7 - 9, and 12 - 19 which are excellent in their antimicrobial activity can be obtained.

The by-product was recrystallized to give (-)-4,5-difluoro-2-methyl-1-[(S)-N-p-toluenesulfonylprolyl]-2,3-dihydroindole (Compound b) having the physical characteristics described below.

mp: 236 - 239°C

$[\alpha]_D^{20}$ = -71.84° (c=1.04, chloroform)

The NMR spectrum of this compound is shown in Fig.2.
The typical peaks are shown in the following.

$^1$H-NMR (DMSO-$d_6$/TMS) δ:

1.36 (3H, d, J=6.15Hz), 1.6 - 2.25 (4H, m), 2.41 (3H, s), 2.83 (1H, d, J=13.5Hz), 3.05 - 3.64 (3H, m), 4.5 - 4.95 (2H, m) 7.41 (2H, d, J=8.35Hz), 7.74 (2H, d, J=8.35Hz), 7.07 - 7.55 (1H, m), 7.6 - 7.9 (1H, m)

Example 2

Synthesis of (+)-4,5-dichloro-2-methyl-1-[(S)-N-p-toluenesulfonyl prolyl]-2,3-dihydroindole

(±)-4,5-dichloro-2-methyl-2,3-dihydroindole was used as the starting substance, and the title compound was obtained in a manner analogous to in Example 1.

white edged crystals, mp: 170 - 173°C

$[\alpha]^{20}_{D}$ = +43.0° (c=0.4, chloroform)

| Elemental analysis for $C_{21}H_{22}Cl_2N_2O_3S$ | | | |
|---|---|---|---|
| Calcd.: | C:55.63, | H:4.86, | N:6.18 |
| Found : | C:55.46, | H:4.78, | N:6.14 |

Example 3

Synthesis of (-)-4,5-difluoro-2-methyl-2,3-dihydroindole p-toluenesulfonate

(-)-4,5-difluoro-2-methyl-1-[(S)-N-p-toluenesulfonylprolyl]-2,3-dihydroindole, the compound (a) obtained in Example 1, 51.7 g, was added to the solution comprising 69 g of potassium hydroxide, 50 mℓ of water and 200 mℓ of methanol, and the mixture was refluxed by heating for 3.5 hours. After the reaction the mixture was allowed to cool to the room temperature, concentrated under reduced pressure to the 1/4 volume, to which was added 800 mℓ of water, and extracted twice with 400 mℓ of ether. The ether layer was washed with 300 mℓ of water and a saturated aqueous solution of sodium chloride, and the organic layer was dried over magnesium sulfate and filtered. To the filtrate was added 24 g of p-toluenesulfonic acid monohydrate, and stirred for 30 minutes. The precipitate was collected by filtration, and recrystallized from ethyl acetate-ethanol, to give 25.8 g of the title compound.

white edged crystals mp: 144 - 147°C

$[\alpha]^{20}_{D}$ = -2.22° (c=1.35, methanol)

| Elemental analysis for $C_{16}H_{17}F_2NO_3S$ | | | |
|---|---|---|---|
| Calcd.: | C:56.29, | H:5.02, | N:4.10 |
| Found : | C:56.13, | H:5.03, | N:4.11 |

Example 4

Synthesis of (-)-4,5-dichloro-2-methyl-2,3-dihydroindole

The compound obtained in Example 2 was used as the starting substance, and treated in a manner analogous to Example 3, to give the title compound.

oily substance

$[\alpha]^{20}_{D}$ = -27.6° (c=1.2, chloroform)

Example 5

Synthesis of (+)-8,9-difluoro-2-methyl-6-oxo-1,2-dihydro-6H-pyrrolo[3,2,1-ij]quinoline-5-carboxylic acid

To 9 g of (-)-4,5-difluoro-2-methyl-2,3-dihydroindole obtained in Example 3 was added 11.5 g of diethylethoxymethylene malonate, and heated at 140 - 150°C for 30 minutes. After cooling, to the reaction mixture was added polyphosphoric acid prepared from 32.1g of phosphoric acid and 32.1 g of phosphorus pentaoxide, and heated at 140 - 150°C for 30 minutes. After the reaction the resultant mixture was cooled to the room temperature, and added to 600 mℓ of ice-water. The mixture was adjusted to pH 3 with a 20% aqueous solution of sodium hydroxide and the precipitating solid was collected by filtration. The solid was added to a mixture comprising 117 mℓ of acetic acid and 30 mℓ of concentrated hydrochloric acid, and refluxed by heating for 5.5 hours. The reaction mixture was added to 1 ℓ of ice-water. The precipitates formed were collected by filtration, washed with water, with isopropanol, and with ether, in this order,

and dried, to give 8.86 g of the title compound.

mp: 279 - 283°C

$[\alpha]^{20}_{D}$ = +26.32° (c=0.57, DMSO)

A part of the product was recrystallized from DMF-$H_2O$.

| Elemental analysis for $C_{13}H_9F_2NO_3$ | | | |
|---|---|---|---|
| Calcd.: | C:58.87, | H:3.42, | N:5.28 |
| Found : | C:58.87, | H:3.15, | N:5.28 |

Example 6

Synthesis of (+)-8,9-dichloro-2-methyl-6-oxo-1,2-dihydro-6H-pyrrolo[3,2,1-ij]quinoline-5-carboxylic acid

The compound obtained in Example 4 was used as the starting substance, and treated in a manner analogous to Example 5, to give the title compound.

mp: 268 - 270°C

$[\alpha]^{20}_{D}$ = +15.5° (c=0.5, 1N-NaOH)

| Elemental analysis for $C_{13}H_9Cl_2NO_3$ | | | |
|---|---|---|---|
| Calcd.: | C:52.35, | H:3.02, | N:4.70 |
| Found : | C:52.24, | H:2.98, | N:4.91 |

Example 7

Synthesis of (+)-8-fluoro-2-methyl-9-(4-methyl-1-piperazinyl)-6-oxo-1,2-dihydro-6H-pyrrolo[3,2,1-ij]quinoline-5-carboxylic acid and the hydrochloride of the same

(+)-8,9-difluoro-2-methyl-6-oxo-1,2-dihydro-6H-pyrrolo[3,2,1-ij]quinoline-5-carboxylic acid obtained in Example 5, 13.5 g, was suspended in 500 mℓ of diethyl ether, to which was added 70 mℓ of boron trifluoride-diethyl ether complex, and stirred at room temperature for 5 hours. After the reaction, the precipitates were collected by filtration, washed with diethyl ether, and dried. The precipitates were dissolved in 100 mℓ of hexamethylphosphoric triamide, to which were added 14.2 mℓ of triethylamine and 7.5 mℓ of N-methylpiperazine, and stirred at 40°C for 10 hours. The solvent was evaporated off under reduced pressure, and to the residue was added diethyl ether. The precipitating yellow solid was collected by filtration. The solid was suspended in 400 mℓ of methanol, to which was added 25 mℓ of triethylamine, and refluxed by heating for 25 hours. The solvent was evaporated off under reduced pressure, to the residue was added 300 mℓ of water, the mixture was adjusted to pH 1 with a 10% aqueous hydrochloric acid, and the insoluble matters were filtered off. The filtrate was concentrated under reduced pressure, and the residue was recrystallized from ethanol-water, to give the hydrochloride of the title compound.

The physical characteristics are described below.

pale yellow edged crystals, mp: 285 - 290°C (decomposed)

$[\alpha]^{20}_{D}$ = +10.70° (c=0.873, 1N-NaOH)

| Elemental analysis for $C_{18}H_{20}FN_3O_3.HCl.H_2O$ | | | |
|---|---|---|---|
| Calcd.: | C:54.07, | H:5.79, | N:10.51 |
| Found : | C:54.31, | H:5.63, | N:10.62 |

Ratio of dissolution to water (at 20°C):230mg/mℓ

The hydrochloride was dissolved in 200 mℓ of water, and the solution was adjusted to pH 11 with a 20% aqueous NaOH and then to pH 7.3 with a 10% aqueous hydrochloric acid. The resultant solution was extracted three times with

2 ℓ of chloroform, and the organic layer was dried with anhydrous sodium sulfate. Chloroform was evaporated off, and the resultant solid was recrystallized from DMF-ethanol, to give 10.5 g of the title compound.

pale yellow edged crystals, mp: 250 - 253°C

$[\alpha]^{20}_{D}$ = +9.24° (c=0.866, DMSO)

| Elemental analysis for $C_{18}H_{20}FNO_3$ | | | |
|---|---|---|---|
| Calcd.: | C:62.60, | H:5.84, | N:12.17 |
| Found : | C:62.40, | H:5.85, | N:12.13 |

Example 8

Synthesis of (+)-8-fluoro-2-methyl-9-(3-methyl-1-piperazinyl)-6-oxo-1,2-dihydro-6H-pyrrolo[3,2,1-ij]quinoline-5-carboxylic acid hydrochloride

(+)-8,9-difluoro-2-methyl-6-oxo-1,2-dihydro-6H-pyrrolo[3,2,1-ij]quinoline-5-carboxylic acid obtained in Example 5, 3 g, was suspended in 30 ml of hexamethylphosphoric triamide, to which was added 3.4 g of 2-methylpiperazine, and allowed to react in an oil bath at 120 - 130°C for 1 hour. The solvent was evaporated off under reduced pressure, to the residue was added ethyl acetate, and the precipitates were collected by filtration. The precipitates were suspended in 150 mℓ of water, the suspension was adjusted to pH 8 with a 1N sodium hydroxide solution and then to pH 1 with a 10% aqueous hydrochloric acid. The water layer was treated with active carbon and concentrated, and the resultant residue was recrystallized from ethanol-water, to give 3.28 g of the title compound.

pale yellow edged crystals, mp: 278 - 283°C (decomposed)

$[\alpha]^{20}_{D}$ = +27.52° (c=0.545, 1N-NaOH)

| Elemental analysis for $C_{18}H_{20}FN_3O_3.HCl.H_2O$ | | | |
|---|---|---|---|
| Calcd.: | C:54.01, | H:5.75, | N:10.50 |
| Found : | C:54.28, | H:5.56, | N:10.55 |

Ratio of dissolution to water (at 20°C): 385 mg/mℓ

$^1$H-NMR (DMSO-$d_6$) δ:

1.30 (d, 3H, J=6Hz), 1.63 (d, 3H, J=6Hz), 2.95 - 4.05 (m, 7H), 4.9 - 5.3 (d, 1H), 7.64 (d, 1H, J=12Hz),8.96 (s, 1H)

In addition, the physical characteristics of (+)-8-fluoro-2-methyl-9-(3-methyl-1-piperazinyl)-6-oxo-1,2-dihydro-6H-pyrrolo [3,2,1-ij]quinoline-5-carboxylic acid are described below.

$[\alpha]^{22}_{D}$ = +25.5 ° (c=1.02, 1N-NaOH)

Ratio of dissolution to water (at 20°C): 143 mg/ml

$^1$H-NMR (DMSO-$d_6$) δ:

1.31 (d, 3H, J=6Hz), 1.65 (d, 3H, J=6Hz), 2.53 (6s, 2H), 2.95 - 4.1 (m), 4.9 - 5.3 (2H, m), 7.63 (d, 1H, J=12Hz), 8.92 (s, 1H)

Example 9

Synthesis of (+)-8-fluoro-2-methyl-9-(3,4-dimethyl-1-piperazinyl)-6-oxo-1,2-dihydro-6H-pyrrolo[3,2,1-ij]quinoline-5-carboxylic acid

To 7 g of (+)-8-fluoro-2-methyl-9-(3-methyl-1-piperazinyl)-6-oxo-1,2-dihydro-6H-pyrrolo[3,2,1-ij]quinoline-5-carboxylic acid obtained in Example 8 were added 36 g of sodium formate, 90 mℓ of 99% formic acid, and 90 mℓ of 37% formalin, and refluxed by heating for 3 hours. After the reaction the mixture was concentrated under reduced pressure, to the residue was added 200 mℓ of water, and the mixture was adjusted to pH 7.5 with a 20% aqueous sodium hydroxide. The mixture was extracted twice with 500 mℓ of chloroform, and the organic layer was washed with a saturated solution of sodium chloride and then with water, and dried with anhydrous sodium sulfate.

Chloroform was evaporated off, and the residue was recrystallized from benzene-n-hexane to give 5.5 g of the title compound.

pale yellow edged crystals, mp: 210 - 212°C (decomposed)
$[\alpha]_D^{20}$ = +10.53° (c=0.57, 1N-NaOH)

| Elemental analysis for $C_{19}H_{22}FN_3O_3$ | | | |
|---|---|---|---|
| Calcd.: | C:63.50, | H:6.17, | N:11.69 |
| Found : | C:63.20, | H:6.17, | N:11.55 |

By treating this compound with 10% aqueous hydrochloric acid (+)-8-fluoro-2-methyl-9-(3,4-dimethyl-1-piperazinyl)-6-oxo-1,2-dihydro-6H-pyrrolo[3,2,1-ij]quinoline-5-carboxylic acid hydrochloride was obtained.
pale yellow edged crystals, mp: 278 - 282°C (decomposed)
$[\alpha]_D^{20}$ = +12.13° (c=0.58, 1N-NaOH)

| Elemental analysis for $C_{19}H_{22}FN_3O_3.H_2O.HCl$ | | | |
|---|---|---|---|
| Calcd.: | C:55.14, | H:6.09, | N:10.15 |
| Found : | C:55.29, | H:5.97, | N:10.34 |

Example 10

(±)-8-fluoro-2-methyl-9-(3-methyl-1-piperazinyl)-6-oxo-1,2-dihydro-6H-pyrrolo[3,2,1-ij]quinoline-5-carboxylic acid hydrochloride

(±)-8,9-difluoro-2-methyl-6-oxo-1,2-dihydro-6H-pyrrolo[3,2,1-ij]quinoline-5-carboxylic acid was used as the starting substance, and treated in a manner analogous to Example 8, to give the title compound.
pale yellow edged crystals, mp: 258 - 261°C (decomposed)

| Elemental analysis for $C_{18}H_{20}FN_3O_3.HCl.H_2O$ | | | |
|---|---|---|---|
| Calcd.: | C:54.01, | H:5.75, | N:10.50 |
| Found : | C:53.86, | H:5.82, | N:10.36 |

Ratio of dissolution to water(at 20°C): 152 mg/mℓ

Example 11

Synthesis of (+)-8-chloro-2-methyl-9-(3-methyl-1-piperazinyl) -6-oxo-1,2-dihydro-6H-pyrrolo[3,2,1-ij]quinoline-5-carboxylic acid hydrochloride

(+)-8,9-dichloro-2-methyl-6-oxo-1,2-dihydro-6H-pyrrolo[3,2,1-ij]quinoline-5-carboxylic acid obtained in Example 6 and 2-methylpiperazine were used as the starting substances, and treated in a manner analogous to Example 8, to give the title compound.
pale yellow edged crystals, mp: 268 - 270°C (decomposed)
$[\alpha]_D^{20}$ = +15.5° (c=0.50, 1N-NaOH)

| Elemental analysis for $C_{18}H_{20}ClN_3O_3.HCl.H_2O$ | | | |
|---|---|---|---|
| Calcd.: | C:51.92, | H:5.53, | N:10.10 |
| Found : | C:51.69, | H:5.42, | N:9.92 |

Ratio of dissolution to water(at 20°C): 231 mg/mℓ

Example 12

Synthesis of (+)-8-fluoro-2-methyl-9-(3,5-dimethyl-1-piperazinyl)-6-oxo-1,2-dihydro-6H-pyrrolo[3,2,1-ij]quinoline-5-carboxylic acid hydrochloride

The compound obtained in Example 5 and 2,6-dimethylpiperazine were used as the starting substances, and treated in a manner analogous to Example 8, to give the title compound.

pale yellow edged crystals, mp: 288 - 295°C (decomposed)

$[\alpha]_D^{20}$ = +10.59° (c=0.85, 1N-NaOH)

| Elemental analysis for $C_{19}H_{23}ClFN_3O_3.2H_2O$ | | | |
|---|---|---|---|
| Calcd.: | C:52.84, | H:6.30, | N:9.73 |
| Found : | C:52.56, | H:5.98, | N:9.47 |

Ratio of dissolution to water (at 20°C): 175 mg/mℓ

Example 13

Synthesis of (+)-8-fluoro-2-methyl-9-(3,4,5-trimethyl-1-piperazinyl)-6-oxo-1,2-dihydro-6H-pyrrolo[3,2,1-ij]quinoline-5-carboxylic acid

The compound obtained in Example 5 and 1,2,6-trimethylpiperazine were used, and treated in a manner analogous to Example 9, to give the title compound.

pale yellow edged crystals, mp: 204 - 206°C

$[\alpha]_D^{20}$ = +16.57° (c=0.905, 1N-NaOH)

| Elemental analysis for $C_{20}H_{24}FN_3O_3$ | | | |
|---|---|---|---|
| Calcd.: | C:64.33, | H:6.48, | N:11.25 |
| Found : | C:64.11, | H:6.24, | N:11.06 |

And (+)-8-fluoro-2-methyl-9-(3,4,5-trimethyl-1-piperazinyl)-6-oxo-1,2-dihydro-6H-pyrrolo[3,2,1-ij]quinoline-5-carboxylic acid hydrochloride was obitained by treating with 10% aqueous hydrochloric acid.

pale yellow sharp-edged crystals mp: 268 - 271°C (decomposed)

$[\alpha]_D^{20}$ = +18.99° (c=0.913, 1N-NaOH)

| Elemental analysis for $C_{20}H_{24}FN_3O_3.HCl.H_2O$ | | | |
|---|---|---|---|
| Calcd.: | C:56.14, | H:6.36, | N:9.82 |
| Found : | C:55.88, | H:6.45, | N:9.71 |

Example 14

Synthesis of (-)-8-fluoro-2-methyl-9-(3-S-methyl-1-piperazinyl)-6-oxo-1,2-dihydro-6H-pyrrolo[3,2,1-ij]quinoline-5-carboxylic acid hydrochloride

(+)-8,9-difluoro-2-methyl-6-oxo-1,2-dihydro-6H-pyrrolo[3,2,1-ij]quinoline-5-carboxylic acid obtained in Example 5 and S-(+)-2-methylpiperazine were used, and treated in a manner analogous to Example 8, to give the title compound.

pale yellow edged crystals, mp: 299°C (decomposed)

$[\alpha]_D^{20}$ = -1.67° (c=0.60, 1N-NaOH)

| Elemental analysis for $C_{18}H_{20}FN_3O_3.HCl.H_2O$ | | | |
|---|---|---|---|
| Calcd.: | C:54.01, | H:5.75, | N:10.50 |
| Found : | C:53.92, | H:5.93, | N:10.24 |

Example 15

Synthesis of (+)-8-fluoro-2-methyl-9-(3-S-4-dimethyl-1-piperazinyl)-6-oxo-1,2-dihydro-6H-pyrrolo[3,2,1-ij]quinoline-5-carboxylic acid

(-)-8-fluoro-2-methyl-9-(3-S-methyl-1-piperazinyl)-6-oxo-1,2-dihydro-6H-pyrrolo[3,2,1-ij]quinoline-5-carboxylic acid obtained in Example 14 was used, and treated in a manner analogous to Example 9, to give the title compound.

pale yellow edged crystals mp: 217 - 219°C

$[\alpha]_D^{20}$ = +2.86° (c=0.35, 1N-NaOH)

| Elemental analysis for $C_{19}H_{22}FN_3O_3$ | | | |
|---|---|---|---|
| Calcd.: | C:63.50, | H:6.17, | N:11.69 |
| Found : | C:63.63, | H:6.05, | N:11.47 |

By treating this with 10% aqueous hydrochloric acid (+)-8-fluoro-2-methyl-9-(3-S-4-dimethyl-1-piperazinyl)-6-oxo-1,2-dihydro-6H-pyrrolo[3,2,1-ij]quinoline-5-carboxylic acid hydrochloride was obtained.

pale yellow edged crystals, mp: 281-283°C (decomposed)

$[\alpha]_D^{20}$ = +3.30° (c=0.36, 1N-NaOH)

| Elemental analysis for $C_{19}H_{22}FN_3O_3.HCl.H_2O$ | | | |
|---|---|---|---|
| Calcd.: | C:55.14, | H:6.09, | N:10.15 |
| Found : | C:54.97, | H:6.22, | N:10.03 |

Example 16

Synthesis of (+)-8-fluoro-2-methyl-9-(3-R-methyl-1-piperazinyl)-6-oxo-1,2-dihydro-6H-pyrrolo[3,2,1-ij]quinoline-5-carboxylic acid hydrochloride

(+)-8,9-Difluoro-2-methyl-6-oxo-1,2-dihydro-6H-pyrrolo[3,2,1-ij]quinoline-5-carboxylic acid obtained in Example 5 and R-(-)-2-methylpiperazine were used, and treated in a manner analogous to Example 8, to give the title compound.

pale yellow edged crystals, mp: 295°C (decomposed)

$[\alpha]_D^{20}$ = +35.29° (c=0.68, 1N-NaOH)

| Elemental analysis for $C_{18}H_{20}FN_3O_3.HCl.H_2O$ | | | |
|---|---|---|---|
| Calcd.: | C:54.01, | H:5.75, | N:10.50 |
| Found : | C:54.17, | H:5.82, | N:10.38 |

Example 17

Synthesis of (+)-8-fluoro-2-methyl-9-(3-methyl-1-piperazinyl)-6-oxo-1,2-dihydro-6H-pyrrolo[3,2,1-ij]quinoline-5-carboxylic acid

To 1 g of (+)-8-fluoro-2-methyl-9-(3-methyl-1-piperazinyl)-6-oxo-1,2-dihydro-6H-pyrrolo[3,2,1-ij]quinoline-5-carboxylic acid obtained in Example 8 was dissolved in 20 mℓ of water, and to which was added 5 g of Bio-Red AG3-X4(OH form)[manufactured by Bio-Red Laboratories]. The reaction was conducted for 10 munites at room temperature. after the reaction, resin was filtered off and the filtrat was concentrated under reduced pressure. To the residue was added 10mℓ of ethanol, to give powder matters.

The residue was filtered and washed with ethanol and n-hexane to give 0.69 g of the title compound.

pale yellow amorphism, mp: over 290°C changing to black

$[\alpha]^{22}_{D}$ = +25.5° (c=1.02, 1N-NaOH)

$^{1}$H-NMR (DMSO-$d_6$) δ :

1.30 (d, 3H, J=6Hz), 1.63 (d, 3H, J=6Hz), 2.95 - 4.05 (m, 7H), 4.9 - 5.3 (m, 1H), 7.64 (d, 1H, J=12Hz), 8.96(s, 1H)

Example 18

Synthesis of (+)-8-fluoro-2-methyl-9-(3-methyl-1-piperazinyl)-6-oxo-1,2-dihydro-6H-pyrrolo[3,2,1-ij]quinoline-5-carboxylic acid 1/2-sulfate

To 0.5 g of (+)-8-fluoro-2-methyl-9-(3-methyl-1-piperazinyl)-6-oxo-1,2-dihydro-6H-pyrrolo[3,2,1-ij]quinoline-5-carboxylic acid obtained in Example 20 was dissolved in 10 mℓ of water, and was adjusted to pH 1 with 1N-sulfulic acid. The water layer was concentrated under reduced pressure, and the residue obitained was recrystallized from ethanol-water, to give 0.41 g of the title compound.

white edged crystals, mp: 262 - 265°C (decomposed)

$[\alpha]^{20}_{D}$ = +27.15° (c=0.551, 1N-NaOH)

| Elemental analysis for $C_{18}N_{20}FN_3O_3.HCl.1/2H_2SO_4$ | | | |
|---|---|---|---|
| Calcd.: | C:54.82, | H:5.37, | N:10.65 |
| Found : | C:54.66, | H:5.31, | N:10.53 |

Example 19

Synthesis of (+)-8-fluoro-2-methyl-9-(3-methyl-1-piperazinyl)-6-oxo-1,2-dihydro-6H-pyrrolo[3,2,1-ij]quinoline-5-carboxylic acid methanesulfonate

(+)-8-fluoro-2-methyl-9-(3-methyl-1-piperazinyl)-6-oxo-1,2-dihydro-6H-pyrrolo[3,2,1-ij]quinoline-5-carboxylic acid and methanesulfonic acid were used, and treated in a manner analogous to Example 21, to give the title compound.

pale yellow edged crystals, mp: 247 - 251°C (decomposed)

$[\alpha]^{20}_{D}$ = +31.62° (c=0.554, 1N-NaOH)

| Elemental analysis for $C_{18}H_{20}FN_3O_3.CH_3.SO_3H.H_2O$ | | | |
|---|---|---|---|
| Calcd.: | C:49.66, | H:5.70, | N:9.14 |
| Found : | C:49.80, | H:5.64, | N:9.08 |

Reference Example 1

Synthesis of (-)-8-fluoro-2-methyl-9-(4-methyl-1-piperazinyl)-6-oxo-1,2-dihydro-6H-pyrrolo[3,2,1-ij]quinoline-5-carboxylic acid

(-)-8,9-difluoro-2-methyl-6-oxo-1,2-dihydro-6H-pyrrolo[3,2,1-ij]quinoline-5-carboxylic acid (Compound b) and N-methylpiperazine were used, and treated in a manner analogous to Example 7, to give the title compound.

pale yellow edged crystals, mp: 249 - 252°C
$[\alpha]^{20}_{D}$ = -8.98° (c=0.806, DMSO)

Reference Example 2

Synthesis of (-)-8-fluoro-2-methyl-9-(3-methyl-1-piperazinyl)-6-oxo-1,2-dihydro-6H-pyrrolo[3,2,1-ij]quinoline-5-carboxylic acid hydrochloride

(-)-8,9-difluoro-2-methyl-6-oxo-1,2-dihydro-6H-pyrrolo[3,2,1-ij]quinoline-5-carboxylic acid and N-methylpiperazine were used, and treated in a manner analogous to Example 8, to give the title compound.
$[\alpha]^{20}_{D}$ = -18.00° (c=1.00, 1N-NaOH)
Ratio of dissolution to water(at 20°C): 167 mg/mℓ
$^1$H-NMR (DMSO-$d_6$) δ :
1.30 (d, 3H, J=6Hz), 1.63 (d, 3H, J=6Hz), 2.95 - 4.05(m, 7H), 4.9 - 5.3(m, 1H), 7.64 (d, 1H, J=12Hz), 8.96(s, 1H)

Reference Example 3

(±)-8-fluoro-2-methyl-9-(4-methyl-1-piperazinyl)-6-oxo-1,2-dihydro-6H-pyrrolo[3,2,1-ij]quinoline-5-carboxylic acid hydrochloride(well-known compound)
Ratio of dissolution to water (at 20°C): 12 mg/mℓ

Pharmacological Activity Test Example 1

In order to investigate the in vitro activity of the under-mentioned compounds against various bacteria, the minimal inhibitory concentration (MIC, μg/mℓ) values were determined by the agar plate dilution method [see Chemotherapy, 22, 1126-1128 (1974)].
The results are shown in Table 1.

Test Compound A

(+)-8-fluoro-2-methyl-9-(4-methyl-1-piperazinyl)-6-oxo-1,2-dilydro-6H-pyrrolo[3,2,1-ij]quinoline-5-carboxylic acid (a (+)-isomer, a compound according to the present invention, Example 7)

Test Compound B

(+)-8-fluoro-2-methyl-9-(3-methyl-1-piperazinyl)-6-oxo-1,2-dihydro-6H-pyrrolo[3,2,1-ij]quinoline-5-carboxylic acid hydrochloride (a compound according to the present invention, Example 8)

Test Compound C

(+)-8-chloro-2-methyl-9-(3-methyl-1-piperazinyl)-6-oxo-1,2-dihydro-6H-pyrrolo[3,2,1-ij]quinoline-5-carboxylic acid hydrochloride (a compound according to the present invention, Example 11)

Test Compound E

8-fluoro-2-methyl-9-(4-methyl-1-piperazinyl)-6-oxo-1,2-dihydro-6H-pyrrolo[3,2,1-ij]quinoline-5-carboxylic acid (racemic body, a reference compound, U.S. Patent 4,416,884.

Test Compound F

(-)-8-fluoro-2-methyl-9-(4-methyl-1-piperazinyl)-6-oxo-1,2-dihydro-6H-pyrrolo[3,2,1-ij]quinoline-5-carboxylic acid (a (-)-isomer, a comparative compound, Reference Example 1)

Test Compound G

S-(-)-9-fluoro-3-methyl-10-(4-methyl-1-piperazinyl)-7-oxo-2,3-dihydro-7H-pyrido[1,2,3-de][1,4]benzoxazine-6-carboxylic acid (ofloxacine, a comparative compound)

Table 1

| Strain | MIC (µg/mℓ) | | | | | |
|---|---|---|---|---|---|---|
| | Test Compound | | | | | |
| | A | B | C | E | F | G |
| S.aureus FDA209P | 0.1 | 0.2 | 0.39 | 0.2 | 3.13 | 0.2 |
| E.facalis ATCC-21212 | 0.78 | 3.13 | 6.25 | 1.56 | 100 | 3.13 |
| E.coli NIHJ JC-2 | 0.05 | 0.05 | 0.1 | 0.1 | 0.78 | 0.1 |
| K.pneumoniae NCTC-9632 | 0.05 | 0.05 | 0.1 | 0.1 | 0.78 | 0.1 |
| S.marcoscons IFO-12648 | 0.05 | 0.1 | 0.2 | 0.1 | 0.78 | 0.2 |
| P.mirabilis ATCC-29906 | 0.2 | 0.1 | 0.2 | 0.39 | 3.13 | 0.39 |
| M.morganii IID Kono | 0.05 | 0.012 | 0.024 | 0.1 | 0.78 | 0.1 |
| P.aeruginosa ATCC-10145 | 1.56 | 0.39 | 0.78 | 3.13 | 25 | 3.13 |
| P.aeruginosa E-2 | 0.78 | 0.39 | 0.78 | 1.56 | 12.5 | 1.50 |
| A.calcoaceticus Ac-54 | 0.1 | 0.39 | 0.78 | 0.2 | 3.13 | 0.39 |

Pharmaceutical Example 1

| | |
|---|---|
| Compound of Example 7 | 2 g |
| Purified lanolin | 5 g |
| White beeswax | 5 g |
| White petrolatum | 88 g |
| Total | 100 g |

White beeswax was melted by warming and, then, the compound according to the present invention, purified lanolin and white petrolatum were added. The mixture was warmed until it formed a liquid and, then, stirred until it was solidified to give an ointment of the above composition.

Pharmaceutical Example 2

| | |
|---|---|
| Compound of Example 8 | 200 mg |
| Glucose | 250 mg |
| Distilled water for injection | q.s. |

In distilled water for injection were dissolved the compound of the present invention and glucose and the solution was filled into a 5 ml ampul. After nitrogen purging, sterilization was carried out by autoclaving at 121°C for 15 minutes to give a parenteral product of the above composition.

Pharmaceutical Example 3

| | |
|---|---|
| Compound of Example 11 | 100 g |
| Avicel [trademark of Asahi Chemical Industry] | 40 g |
| Corn starch | 30 g |
| Magnesium stearate | 2 g |
| TC-5 [trademark of Shinetsu Chemical: hydroxypropylmethylcellulose] | 10 g |
| Macrogol-6000 | 3 g |
| Castor oil | 40 g |
| Methanol | 40 g |

The compound according to the present invention, avicel, corn starch and magnesium stearate were milled together and tableted by means of R 10 mm punch (for sugar-coated tablets).
The resulting tablets were coated with a film coating composition consisting of TC-5, polyethylene glycol (macrogol-6000), castor oil and methanol to give film-coated tablets of the above composition.

**Claims**

1. A pyrroloquinoline derivative of the general formula I:

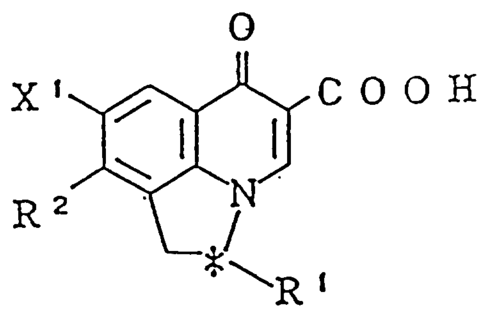

(I)

wherein $R^1$ is a $C_1$-$C_6$ alkyl group; $R^2$ is a group:

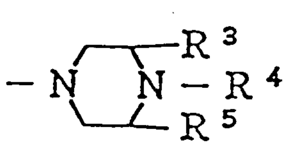

wherein $R^3$, $R^4$ and $R^5$ are the same or different, a hydrogen atom or a $C_1$-$C_6$ alkyl group, provided that at least one of the groups of $R^3$, $R^4$ and $R^5$ is the $C_1$-$C_6$ alkyl group, $X^1$ is a halogen atom and * denotes an asymmetric atom, which has optical activity due to the asymmetric carbon atom at the 2-position wherein the 2-position asymmetric carbon atom of pyrroloquinoline ring has the same configuration as the 2-position asymmetric carbon atom of one of the two diastereoisomers of (4,5-difluoro-2-methyl-1-[(S)-N-p-toluenesulfonylprolyl]-2,3-dihydroindole which show an $[a]_D^{20}$ = - 10.38° (c = 1.06, chloroform)), or salt thereof.

2. A pyrroloquinoline derivative of salt thereof according to claim 1, wherein $R^2$ is a group:

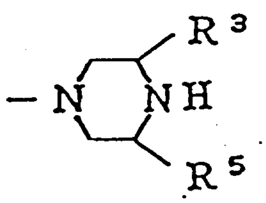

wherein $R^3$ and $R^5$ are the same or different, a hydrogen atom or a $C_1$-$C_6$ alkyl group, provided that at least one of the groups of $R^3$ and $R^5$ is the $C_1$-$C_6$ alkyl group.

3. A pyrroloquinoline derivative or salt thereof according to claim 1, wherein $R^2$ is a group:

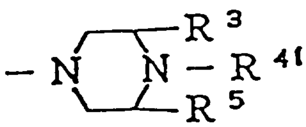

wherein $R^3$ and $R^5$ are the same or different, a hydrogen atom or a $C_1$-$C_6$ alkyl group, and $R^{4'}$ is a $C_1$-$C_6$ alkyl group.

4. A pyrroloquinoline derivative or salt thereof according to claim 2, wherein alkyl group is methyl group or ethyl group and $X^1$ is a fluorine atom or a chlorine atom.

5. A pyrroloquinoline derivative or salt thereof according to claim 4, wherein $X^1$ is a fluorine atom.

6. A pyrroloquinoline derivative or salt thereof according to claim 4, wherein alkyl group is methyl group, and $X^1$ is a chlorine atom.

7. A pyrroloquinoline derivative or salt thereof according to claim 5, wherein alkyl group is methyl group.

8. A pyrroloquinoline derivative salt according to claim 7.

9. A pyrroloquinoline derivative, which is not salt, according to claim 7.

10. A pyrroloquinoline derivative salt according to claim 6.

11. A pyrroloquinoline derivative, which is not salt, according to claim 6.

12. A pyrroloquinoline derivative salt according to claim 3.

13. A pyrroloquinoline derivative, which is not salt, according to claim 3.

14. A pyrroloquinoline derivative or salt thereof according to claim 12, wherein alkyl group is methyl group, and $X^1$ is a fluorine atom.

15. A pyrroloquinoline derivative or salt thereof according to claim 13, wherein alkyl group is methyl group, and $X^1$ is a fluorine atom.

16. A pyrroloquinoline derivative or salt thereof according to claim 14, wherein one of $R^3$ and $R^5$ is a hydrogen atom.

17. A pyrroloquinoline derivative or salt thereof according to claim 14, wherein $R^3$ and $R^5$ are $C_1$-$C_6$ alkyl groups.

**18.** A pyrroloquinoline derivative of the general formula III:

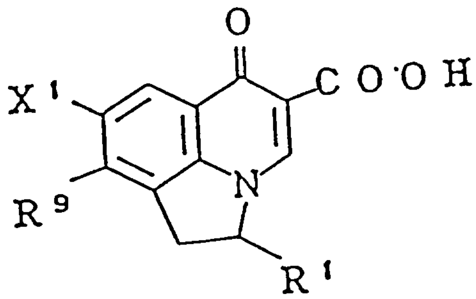

(III)

wherein $R^1$ is a $C_1$-$C_6$ alkyl group; $R^9$ is a group:

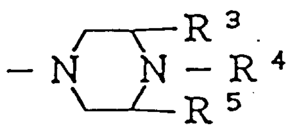

wherein $R^3$ is a $C_1$-$C_6$ alkyl group, and $R^4$ and $R^5$ are hydrogen atoms. $X^1$ is a halogen atom or a salt thereof which is a racemic modification.

**19.** A pyrroloquinoline derivative or salt thereof according to claim 18, wherein $X^1$ is a fluorine atom, $R^1$ is methyl group, and $R^9$ is a group:

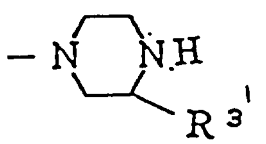

wherein $R^{3'}$ is methyl group or ethyl group.

**20.** An optically active compound of the general formula II:

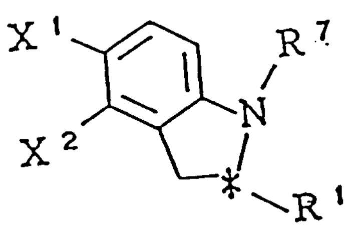

(II)

wherein $R^1$ is a $C_1$-$C_6$ alkyl group, $R^7$ is a hydrogen atom or a group:

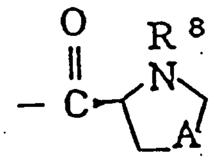

wherein $R^8$ is a protective group, selected from $C_1C_6$ alkyl-substituted phenylsulfonyl groups and $C_1$-$C_6$ alkyl-substituted phenylcarbonyl groups. A is a $C_1$-$C_4$ alkylene group, $X^1$ and $X^2$ are halogen atoms, and * denotes an asymmetric atom which has optical activity due to the asymmetric carbon atom of 2-position, which has the same configuration as the 2-position asymmetric carbon atom of one of the two diastereoisomers of 4,5-difluoro-2-methyl-1-[(S)-N-p-toluenesulfonyl-propyl]-2,3-dihydroindole which show $[\alpha]_D^{20}$ = - 10.38° (c = 1.06 chloroform).

**21.** A pyrroloquinoline derivative of the general formula IV:

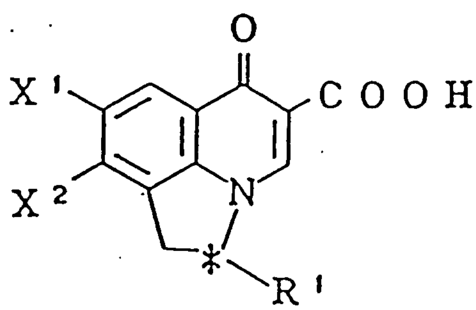

(IV)

wherein $R^1$ is $C_1$-$C_6$ alkyl group, $X^1$ and $X^2$ are halogen atoms, and * denotes an asymmetric carbon atom, wherein the 2-position asymmetric carbon atom of pyrroloquinoline ring has the same configuration as the 2-position asymmetric carbon atom of one of the two diastereoisomers of 4,5-difluoro-2-methyl-1-[(S)-N-p-toluenesulfonylprolyl]-2,3-dihydroindole which show $[\alpha]_D^{20}$ = - 10.38° (c = 1.06, chloroform)), or salt thereof.

**22.** (+)-8-fluoro-2-methyl-9-(3-methyl-1-piperazinyl)-6-oxo-1,2-dihydro-6H-pyrrolo[3,2,1-ij]quinoline-5-carboxylic acid hydrochloride ($[\alpha]^{20}$ = + 27.52° (c = 0.545, 1N NaOH)), (±)-8-fluoro-2-methyl-9-(3-methyl-1-piperazinyl)-6-oxo-1,2-dihydro-6H-pyrrolo[3,2,1-ij]quinoline-5-carboxylic acid hydrochloride (+)-8-chloro-2-methyl-9-(3-methyl-1-piperazinyl)-6-oxo-1,2-dihydro-6H-pyrrolo[3,2,1-ij]quinoline-5-carboxylic acid hydrochloride ($[\alpha]^{20}$ = + 15.5° (c = 0.50, 1N NaOH)), (+)-8-fluoro-2-methyl-9-(3,5-dimethyl-1-piperazinyl)-6-oxo-1,2-dihydro-6H-pyrrolo[3,2,1-ij]quinoline-5-carboxylic acid hydrochloride ($[\alpha]^{20}$ = + 10.59° (c = 0.85, 1N NaOH)), (-)-8-fluoro-2-methyl-9-(3-S-methyl-1-piperazinyl)-6-oxo-1,2-dihydro-6H-pyrrolo[3,2,1-ij]quinoline-5-carboxylic acid hydrochloride ($[\alpha]^{20}$ = -1.67° (c = 0.60, 1N NaOH)), or (+)-8-fluoro-2-methyl-9-(3-R-methyl-1-piperazinyl)-6-oxo-1,2-dihydro-6H-pyrrolo[3,2,1-ij]quinoline-5-carboxylic acid hydrochloride ($[\alpha]^{20}$ = + 35.29° (c = 0.68, 1N NaOH)) according to claim 4.

**23.** (+)-8-fluoro-2-methyl-9-(4-methyl-1-piperazinyl)-6-oxo-1,2-dihydro-6H-pyrrolo[3,2,1-ij]quinoline-5-carboxylic acid ($[\alpha]^{20}$ = + 9.24° (c = 0.866, DMSO)), (+)-8-fluoro-2-methyl-9-(4-methyl-1-piperazinyl)-6-oxo-1,2-dihydro-6H-pyrrolo[3,2,1-ij]quinoline-5-carboxylic acid hydrochloride ($[\alpha]^{20}$ = + 10.70° (c = 0.873, 1N Na OH)), (+)-8-fluoro-2-methyl-9-(3,4-dimethyl-1-piperazinyl)-6-oxo-1,2-dihydro-6H-pyrrolo[3,2,1,ij]quinoline- 5-carboxylic acid ($[\alpha]^{20}$ = + 10.53° (c = 0.57, 1N NaOH)), (+)-8-fluoro-2-methyl-9-(3,4-dimethyl-1-piperazinyl)-6-oxo-1,2-dihydro-6H-pyrrolo[3,2,1-ij]quinoline-5-carboxylic acid hydrochloride ($[\alpha]^{20}$ = + 12.13° (c = 0.58, 1N NaOH)), (+)-8-fluoro-2-methyl-9-(3,4,5-trimethyl-1-piperazinyl)-6-oxo-1,2-dihydro-6H-pyrrolo[3,2,1-ij]quinoline-5-carboxylic acid ($[\alpha]^{20}$ = + 16.57° (c = 0.905, 1N NaOH)), (+)-8-fluoro-2-methyl-9-(3,4,5-trimethyl-1-piperazinyl)-6-oxo-1,2-dihydro-6H-pyrrolo[3,2,1-ij]quinoline-5-carboxylic acid hydrochloride ($[\alpha]^{20}$ = + 18.99° (c = 0.913, 1N NaOH)), (+)-8-fluoro-2-methyl-9-((3S)-3,4-dimethyl-1-piperazinyl)-6-oxo-1,2-dihydro-6H-pyrrolo[3,2,1-ij]quinoline-5-carboxylic acid ($[\alpha]_D^{20}$ = + 2.86° (c = 0.35, 1N

NaOH)), or (+)-8-fluoro-2-methyl-9-((3S)-3,4-dimethyl-1-piperazinyl)-6-oxo-1,2-dihydro-6H-pyrrolo[3,2,1-j]quinoline-5-carboxylic acid hydrochloride ($[\alpha]^{20}$ = + 3.30° (c = 0.36, 1N NaOH)), according to claim 5.

**24.** (+)-8,9-difluoro-2-methyl-6-oxo-1,2-dihydro-6H-pyrrolo[3,2,1-ij]quinoline-5-carboxylic acid ($[\alpha]^{20}$ = + 26.32° (c = 0.57, DMSO)) according to claim 21.

**25.** (+)-8-fluoro-2-methyl-9-(3-methyl-1-piperazinyl)-6-oxo-1,2-dihydro-6H-pyrrolo[3,2,1-ij]quinoline-5-carboxylic acid hydrochloride ($[\alpha]^{20}$ = + 27.52° (c = 0.545, 1N NaOH)), (-)-8-fluoro-2-methyl-9-(3-S-methyl-1-piperazinyl)-6-oxo-1,2-dihydro-6H-pyrrolo[3,2,1-ij]quinoline-5-carboxylic acid hydrochloride ($[\alpha]^{20}$ = - 1.67° (c = 0.60, 1N NaOH)) or (+)-8-fluoro-2-methyl-9-(3-R-methyl-1-piperazinyl)-6-oxo-1,2-dihydro-6H-pyrrolo[3,2,1-ij]quinoline-5-carboxylic acid hydrochloride ($[\alpha]^{20}$ = + 35.29° (c = 0.68, 1N NaOH)) according to claim 22.

**26.** (+)-8-fluoro-2-methyl-9-(3,4-dimethyl-1-piperazinyl)-6-oxo-1,2-dihydro-6H-pyrrolo[3,2,1,ij]quinoline-5-carboxylic acid ($[\alpha]^{20}$ = + 10.53° (c = 0.57, 1N NaOH)), (+)-8-fluoro-2-methyl-9-(3,4-dimethyl-1-piperazinyl)-6-oxo-1,2-dihydro-6H-pyrrolo[3,2,1,ij]quinoline-5-carboxylic acid hydrochloride ($[\alpha]^{20}$ = + 12.13° (c = 0.58, 1N NaOH)), (+)-8-fluoro-2-methyl-9-((3S)-3,4-dimethyl-1-piperazinyl)-6-oxo-1,2-dihydro-6H-pyrrolo[3,2,1,ij]quinoline-5-carboxylic acid ($[\alpha]_D^{20}$ = + 2.86° (c = 0.35, 1N NaOH)), or (+)-8-fluoro-2-methyl-9-((3S)-3,4-dimethyl-1-piperazinyl)-6-oxo-1,2-dihydro-6H-pyrrolo[3,2,1,ij]quinoline-5-carboxylic acid hydrochloride ($[\alpha]_D^{20}$ = + 3.30° (c = 0.36, 1N NaOH)) according to claim 23.

**27.** A process for preparing a pyrroloquinoline derivative according to claim 1 or 21, which comprises:

a) reacting a compound represented by general formula (1):

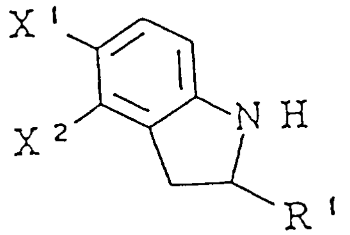

(1)

wherein $R^1$ is a $C_1$-$C_6$ alkyl group, $X^1$ and $X^2$ are halogen atoms, with a compound represented by general formula (2):

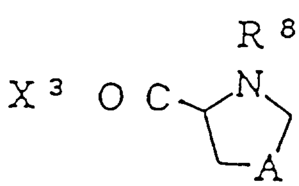

(2)

wherein $R^8$ is a protective group, A is a $C_1$-$C_4$ alkylene group, $X^3$ is a hydroxy group, a halogen atom or a reactive derivative residue of carboxyl group, to obtain a compound represented by general formula (3):

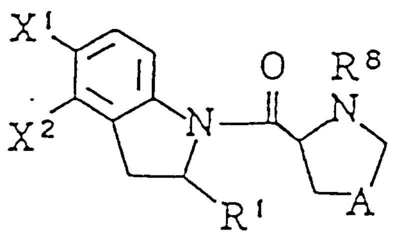

(3)

wherein $R^1$, $R^8$, $X^1$, $X^2$ and A are the same meanings as mentioned above,

b) separating an optically active compound represented by general formula (IIa):

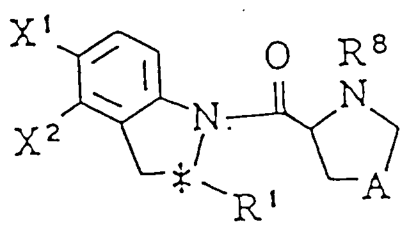

(IIa)

from said compound (3), and hydrolizing the optically active compound (IIa) to obtain a compound represented by general formula (IIb):

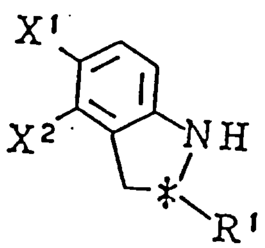

(IIb)

wherein $R^1$, $X^1$ and $X^2$ have the same meanings as mentioned above, and * has the same meanings as defined in claim 1,

c) reacting said compound (IIb) with dialkylethoxymethylenmalonate to obtain a compound represented by general formula (4):

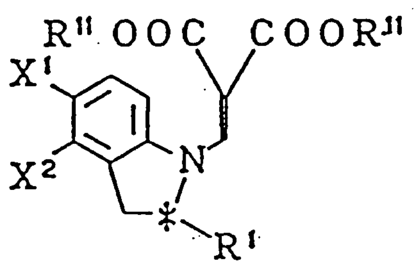

(4)

wherein $R^1$, $X^1$ and $X^2$ have the same meanings as mentioned above, $R^{11}$ is $C_1$-$C_6$ alkyl group, and * has the same meanings as defined in claim 1,

d) cyclizing and hydrolizing said compound (4) to obtain a compound represented by general formula (IV):

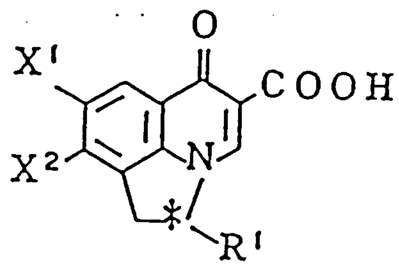

(IV)

wherein $R^1$, $X^1$ and $X^2$ have the same meanings as mentioned above, and * has the same meanings as defined in claim 1.

e) reacting said compound (IV) with the compound represented by general formula (5):

$R^2$ H (5)

wherein $R^2$ is group:

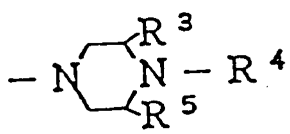

wherein $R^3$, $R^4$ and $R^5$ are the same or different, a hydrogen atom or a $C_1$-$C_6$ alkyl group, provided that at least one of the groups of $R^3$, $R^4$ and $R^5$ is the $C_1$-$C_6$ alkyl group, to obtain a compound represented by a general formula (I):

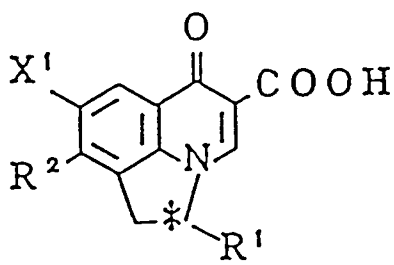

(I )

wherein $R^1$, $R^2$ and $X^1$ have the same meanings as mentioned above, and * has the same meanings as defined in claim 1,

f) forming a chelate compound represented by general formula (6):

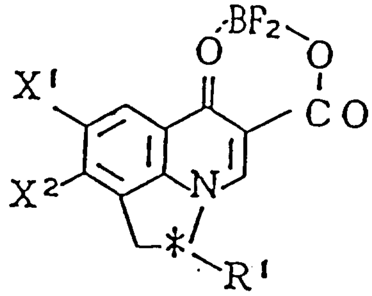

(6)

wherein $R^1$, $X^1$, $X^2$ have the same meanings as mentioned above, and * has the same meanings as defined in claim 1, from said compound (4) or (IV), and then reacting the chelate compound (6) with said compound (5) to obtain said compound (I);

g) reacting said compound (1), which is racemate, with an optically active compound to form a salt of compound (1), and then accomplishing fractional crystallization and desalting to obtain said optically active compound (IIb).

**28.** An antimicrobial agent containing, as an active ingredient, a pyrroloquinoline derivative or salt thereof as defined in claim 1.

**29.** An antimicrobial agent containing, as an active ingredient, a pyrroloquinoline derivative or salt thereof as defined in claim 18.

**30.** An antimicrobial agent containing, as an active ingredient, a pyrroloquinoline derivative or salt thereof as defined in claim 2.

**31.** An antimicrobial agent containing, as an active ingredient, a pyrroloquinoline derivative or salt thereof as defined in claim 3.

**32.** An antimicrobial agent containing, as an active ingredient, a pyrroloquinoline derivative or salt thereof as defined in claim 25.

**33.** An antimicrobial agent containing, as an active ingredient, a pyrroloquinoline derivative or salt thereof as defined in claim 26.

**34.** The use of a pyrroloquinoline derivative or salt thereof as defined in claim 1, 18 or 21 for preparing an antimicrobial composition for human beings and animals.

**Patentansprüche**

**1.** Pyrrolochinolin-Derivat der allgemeinen Formel I:

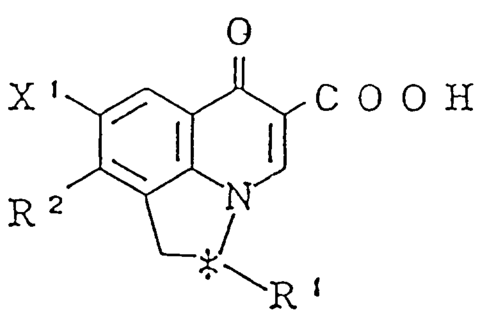

(I)

worin $R^1$ eine $C_1$-$C_6$-Alkyl-Gruppe; $R^2$ eine Gruppe sind:

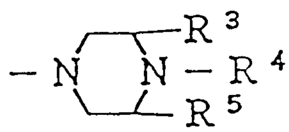

worin $R^3$, $R^4$ oder $R^5$ gleich oder verschieden und ein Wasserstoffatom oder eine $C_1$-$C_6$-Alkyl-Gruppe sind, vorausgesetzt, daß zumindest eine der Gruppen von $R^3$, $R^4$ und $R^5$ die $C_1$-$C_6$-Alkyl-Gruppe ist, worin $X^1$ ein Halogenatom und * ein asymmetrisches Atom sind, das eine optische Aktivität aufgrund des asymmetrischen Kohlenstoffatoms an der 2-Position hat, worin das asymmetrische Kohlenstoffatom der Position 2 die gleiche Konfiguration hat wie das asymmetrische Kohlenstoffatom der Position 2 von einem der beiden Diastereoisomeren von (4,5-Difluor-2-methyl-1-[(S)-N-p-toluolsulfonylprolyl]-2,3-dihydroindol, das einen Wert von $[\alpha]_D^{20}$ = -10,38° (c = 1,06, Chloroform) zeigt, oder ein Salz davon.

2. Pyrrolochinolin-Derivat oder ein Salz davon nach Anspruch 1, worin $R^2$ eine Gruppe ist:

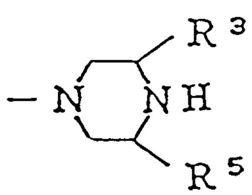

worin $R^3$ und $R^5$ gleich oder verschieden sind und ein Wasserstoffatom oder eine $C_1$-$C_6$-Alkyl-Gruppe bedeuten, vorausgesetzt, daß zumindest eine der Gruppen von $R^3$ und $R^5$ die $C_1$-$C_6$-Alkyl-Gruppe ist.

3. Pyrrolochinolin-Derivat oder ein Salz davon nach Anspruch 1, worin $R^2$ eine Gruppe ist:

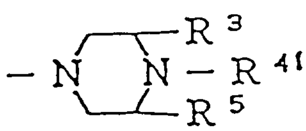

worin $R^3$ und $R^5$ gleich oder verschieden sind, und ein Wasserstoffatom oder eine $C_1$-$C_6$-Alkyl-Gruppe bedeuten und worin $R^{4'}$ eine $C_1$-$C_6$-Alkyl-Gruppe ist.

4. Pyrrolochinolin-Derivat oder ein Salz davon nach Anspruch 2, worin die Alkyl-Gruppe eine Methyl-Gruppe oder Ethyl-Gruppe ist und $X^1$ ein Fluoratom oder ein Chloratom ist.

5. Pyrrolochinolin-Derivat oder ein Salz davon nach Anspruch 4, worin $X^1$ ein Fluoratom ist.

6. Pyrrolochinolin-Derivat oder ein Salz davon nach Anspruch 4, worin die Alkyl-Gruppe eine Methyl-Gruppe und $X^1$ ein Chloratom sind.

7. Pyrrolochinolin-Derivat oder ein Salz davon nach Anspruch 5, worin die Alkyl-Gruppe eine Methyl-Gruppe ist.

8. Pyrrolochinolin-Derivatsalz nach Anspruch 7.

9. Pyrrolochinolin-Derivat nach Anspruch 7, das nicht das Salz ist.

10. Pyrrolochinolin-Derivatsalz nach Anspruch 6.

**11.** Pyrrolochinolin-Derivat nach Anspruch 6, das nicht das Salz ist.

**12.** Pyrrolochinolin-Derivatsalz nach Anspruch 3.

**13.** Pyrrolochinolin-Derivat nach Anspruch 3, das nicht das Salz ist.

**14.** Pyrrolochinolin-Derivat oder Salz davon nach Anspruch 12, worin die Alkyl-Gruppe eine Methyl-Gruppe und $X^1$ ein Fluoratom sind.

**15.** Pyrrolochinolin-Derivat oder ein Salz davon nach Anspruch 13, worin die Alkyl-Gruppe eine Methyl-Gruppe und $X^1$ ein Fluoratom sind.

**16.** Pyrrolochinolin-Derivat oder Salz davon nach Anspruch 14, worin eines von $R^3$ und $R^5$ ein Wasserstoffatom ist.

**17.** Pyrrolochinolin-Derivat oder Salz davon nach Anspruch 14, worin $R^3$ und $R^5$ $C_1$-$C_6$-Alkyl-Gruppen sind.

**18.** Pyrrolochinolin-Derivat der allgemeinen Formel III:

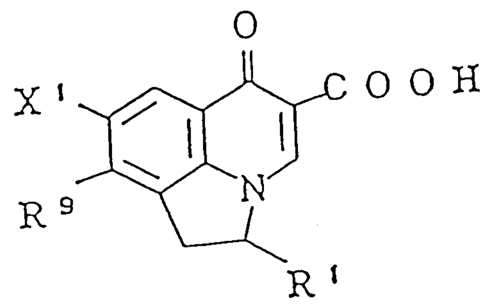

(III)

worin $R^1$ eine $C_1$-$C_6$-Alkyl-Gruppe; $R^9$ eine Gruppe:

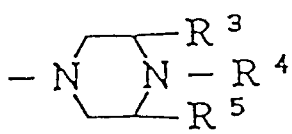

worin $R^3$ eine $C_1$-$C_6$-Alkyl-Gruppe und $R^4$ und $R^5$ Wasserstoffatome, $X^1$ ein Halogenatom sind oder ein Salz davon, das eine racemische Modifizierung ist.

**19.** Pyrrolochinolin-Derivat oder Salz davon nach Anspruch 18, worin $X^1$ ein Fluoratom, $R^1$ eine Methyl-Gruppe und $R^9$ eine Gruppe sind

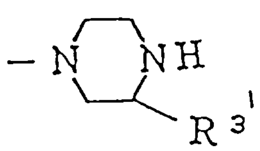

worin $R^{3'}$ Methyl-Gruppe oder Ethyl-Gruppe ist.

**20.** Optisch aktive Verbindung der allgemeinen Formel II:

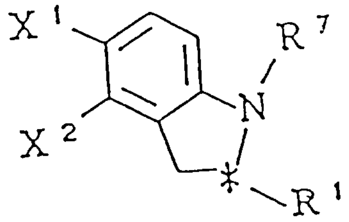

(II)

worin $R^1$ eine $C_1$-$C_6$-Alkyl-Gruppe, $R^7$ ein Wasserstoffatom oder eine Gruppe sind:

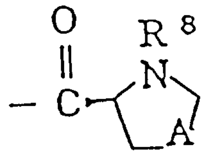

worin $R^8$ eine Schutzgruppe, ausgewählt aus $C_1$-$C_6$-Alkyl-substituierten Phenylsulfonyl-Gruppen und $C_1$-$C_6$-Alkyl-substituierten Phenylcarbonyl-Gruppen, A eine $C_{1-4}$-Alkylen-Gruppe, $X^1$ und $X^2$ Halogenatome und * ein asymmetrisches Atom sind, die eine optische Aktivität aufgrund des asymmetrischen Kohlenstoffatoms an der 2-Position hat, das die gleiche Konfiguration an dem asymmetrischen Kohlenstoffatom der 2-Position von einem der beiden Diastereoisomeren von 4,5-Difluor-2-methyl-1-[(S)-N-p-toluolsulfonyl-prolyl]-2,3-dihydroindol hat, das $[\alpha]_D^{20}$ = -10,38° (c = 1,06 Chloroform) zeigt.

**21.** Pyrrolochinolin-Derivat der allgemeinen Formel IV:

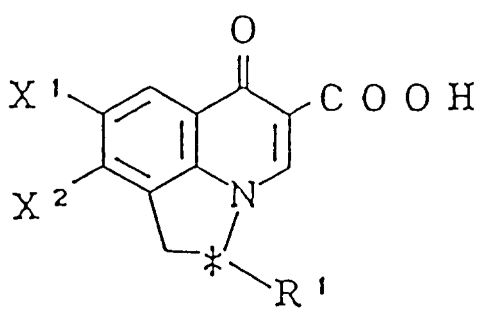

(IV)

worin $R^1$ eine $C_1$-$C_6$-Alkyl-Gruppe, $X^1$ und $X^2$ Halogenatome und * ein asymmetrisches Kohlenstoffatom sind, worin das asymmetrische Kohlenstoffatom der Position 2 des Pyrrolochinolin-Rings die gleiche Konfiguration wie das asymmetrische Kohlenstoffatom an der 2-Position von einem der beiden Diastereoisomeren von 4,5-Difluor-2-methyl-1-[(S)-N-p-toluolsulfonylprolyl]-2,3-dihydroindol hat, das $[\alpha]_D^{20}$ = -10,38° (c = 1,06, Chloroform)) zeigt, oder ein Salz davon.

**22.** (+)-8-Fluor-2-methyl-9-(3-methyl-1-piperazinyl)-6-oxo-1,2-dihydro-6H-pyrrolo[3,2,1-ij]chinolin-5-carbonsäurehydrochlorid ($[\alpha]_D^{20}$ = +27,52° (c = 0,545, 1N NaOH)),

(±)-8-Fluor-2-methyl-9-(3-methyl-1-piperazinyl)-6-oxo-1,2-dihydro-6H-pyrrolo[3,2,1-ij]chinolin-5-carbonsäurehydrochlorid,

(+)-8-Chlor-2-methyl-9-(3-methyl-1-piperazinyl)-6-oxo-1,2-dihydro-6H-pyrrolo[3,2,1-ij]chinolin-5-carbonsäurehydrochlorid ($[\alpha]_D^{20}$ = +15,5° (c = 0,50, 1N NaOH)),

(+)-8-Fluor-2-methyl-9-(3,5-dimethyl-1-piperazinyl)-6-oxo-1,2-dihydro-6H-pyrrolo-[3,2,1-ij]chinolin-5-carbonsäurehydrochlorid ($[\alpha]_D^{20}$ = +10,59° (c = 0,85, 1N NaOH)),
(-)-8-Fluor-2-methyl-9-(3-S-methyl-1-piperazinyl)-6-oxo-1,2-dihydro-6H-pyrrolo[3,2,1-ij]chinolin-5-carbonsäurehydrochlorid ($[\alpha]_D^{20}$ = -1,67° (c = 0,60, 1N NaOH)), oder
(+)-8-Fluor-2-methyl-9-(3-R-methyl-1-piperazinyl)-6-oxo-1,2-dihydro-6H-pyrrolo[3,2,1-ij]chinolin-5-carbonsäurehydrochlorid ($[\alpha]_D^{20}$ = +35,29° (c = 0,68, 1N NaOH)) nach Anspruch 4.

**23.** (+)-8-Fluor-2-methyl-9-(4-methyl-1-piperazinyl)-6-oxo-1,2-dihydro-6H-pyrrolo[3,2,1-ij]chinolin-5-carbonsäure ($[\alpha]_D^{20}$ = +9,24° (c = 0,866, DMSO)),
(+)-8-Fluor-2-methyl-9-(4-methyl-1-piperazinyl)-6-oxo-1,2-dihydro-6H-pyrrolo[3,2,1-ij]chinolin-5-carbonsäurehydrochlorid ($[\alpha]_D^{20}$ = +10,70° (c = 0,873, 1N NaOH)),
(+)-8-Fluor-2-methyl-9-(3,4-dimethyl-1-piperazinyl)-6-oxo-1,2-dihydro-6H-pyrrolo[3,2,1-ij]chinolin-5-carbonsäure ($[\alpha]_D^{20}$ = +10,53° (c = 0,57, 1N NaOH)),
(+)-8-Fluor-2-methyl-9-(3,4-dimethyl-1-piperazinyl)-6-oxo-1,2-dihydro-6H-pyrrolo[3,2,1-ij]chinolin-5-carbonsäurehydrochlorid ($[\alpha]_D^{20}$ = +12,13° (c = 0,58, 1N NaOH)),
(+)-8-Fluor-2-methyl-9-(3,4,5-trimethyl-1-piperazinyl)-6-oxo-1,2-dihydro-6H-pyrrolo[3,2,1-ij]chinolin-5-carbonsäure ($[\alpha]_D^{20}$ = +16,57° (c = 0,905, 1N NaOH)),
(+)-8-Fluor-2-methyl-9-(3,4,5-trimethyl-1-piperazinyl)-6-oxo-1,2-dihydro-6H-pyrrolo[3,2,1-ij]chinolin-5-carbonsäurehydrochlorid ($[\alpha]_D^{20}$ = +18,99° (c = 0,913, 1N NaOH)),
(+)-8-Fluor-2-methyl-9-((3S)-3,4-dimethyl-1-piperazinyl)-6-oxo-1,2-dihydro-6H- pyrrolo[3,2,1-ij]chinolin-5-carbonsäure ($[\alpha]_D^{20}$ = +2,86° (c = 0,35, 1N NaOH)), oder
(+)-8-Fluor-2-methyl-9-((3S)-3,4-dimethyl-1-piperazinyl)-6-oxo-1,2-dihydro-6H-pyrrolo[3,2,1-ij]chinolin-5-carbonsäurehydrochlorid ($[\alpha]_D^{20}$ = +3,30° (c = 0,36, 1N NaOH)), nach Anspruch 5.

**24.** (+)-8,9-Difluor-2-methyl-6-oxo-1,2-dihydro-6H-pyrrolo[3,2,1-ij]chinolin-5-carbonsäure ($[\alpha]_D^{20}$ = +26,32° (c = 0,57, DMSO)), nach Anspruch 21.

**25.** (+)-8-Fluor-2-methyl-9-(3-methyl-1-piperazinyl)-6-oxo-1,2-dihydro-6H-pyrrolo[3,2,1-ij]chinolin-5-carbonsäurehydrochlorid ($[\alpha]_D^{20}$ = +27,52° (c = 0,545, 1N NaOH)),
(-)-8-Fluor-2-methyl-9-(3-S-methyl-1-piperazinyl)-6-oxo-1,2-dihydro-6H-pyrrolo[3,2,1-ij]chinolin-5-carbonsäurehydrochlorid ($[\alpha]_D^{20}$ = -1,67° (c = 0,60, 1N NaOH)), oder
(+)-8-Fluor-2-methyl-9-(3-R-methyl-1-piperazinyl)-6-oxo-1,2-dihydro-6H-pyrrolo[3,2,1-ij]chinolin-5-carbonsäurehydrochlorid ($[\alpha]_D^{20}$ = +35,29° (c = 0,68, 1N NaOH)), nach Anspruch 22.

**26.** (+)-8-Fluor-2-methyl-9-(3,4-dimethyl-1-piperazinyl)-6-oxo-1,2-dihydro-6H-pyrrolo[3,2,1-ij]chinolin-5-carbonsäure ($[\alpha]_D^{20}$ = +10,53° (c = 0,57, 1N NaOH),
(+)-8-Fluor-2-methyl-9-(3,4-dimethyl-1-piperazinyl)-6-oxo-1,2-dihydro-6H-pyrrolo[3,2,1-ij]chinolin-5-carbonsäurehydrochlorid ($[\alpha]_D^{20}$ = +12,13° (c = 0,58, 1N NaOH)),
(+)-8-Fluor-2-methyl-9-((3S)-3,4-dimethyl-1-piperazinyl)-6-oxo-1,2-dihydro-6H- pyrrolo[3,2,1-ij]chinolin-5-carbonsäure ($[\alpha]_D^{20}$ = +2,86° (c = 0,35, 1N NaOH)), oder
(+)-8-Fluor-2-methyl-9-((3S)-3,4-dimethyl-1-piperazinyl)-6-oxo-1,2-dihydro-6H-pyrrolo[3,2,1-ij]chinolin-5-carbonsäurehydrochlorid ($[\alpha]_D^{20}$ = +3,30° (c = 0,36, 1N NaOH)) nach Anspruch 23.

**27.** Verfahren zur Herstellung eines Pyrrolochinolin-Derivates nach Anspruch 1 oder 21, umfassend:

a) Reaktion einer Verbindung, dargestellt durch die allgemeine Formel (1):

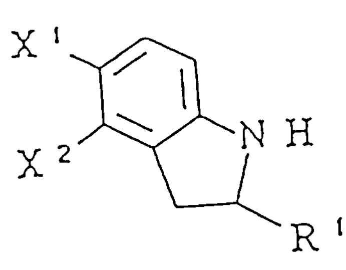

(1)

worin $R^1$ eine $C_1$-$C_6$-Alkyl-Gruppe, $X^1$ und $X^2$ Halogenatome sind, mit einer Verbindung, dargestellt durch die

allgemeine Formel (2):

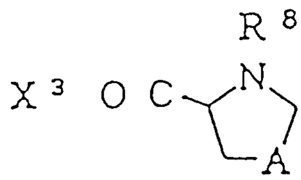

(2)

worin $R^8$ eine Schutzgruppe, A eine $C_1$-$C_4$-Alkylen-Gruppe, $X^3$ eine Hydroxy-Gruppe, ein Halogenatom oder ein reaktiver Derivatrest von Carboxyl-Gruppe sind, unter Erhalt einer Verbindung, dargestellt durch die allgemeine Formel (3):

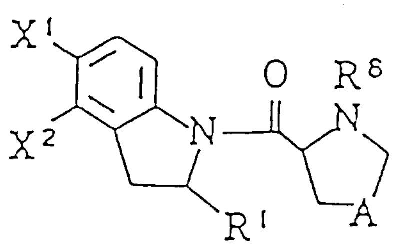

(3)

worin $R^1$, $R^8$, $X^1$, $X^2$ und A die gleichen Bedeutungen wie oben haben,

b) Trennung einer optisch aktiven Verbindung, dargestellt durch die allgemeine Formel (IIa):

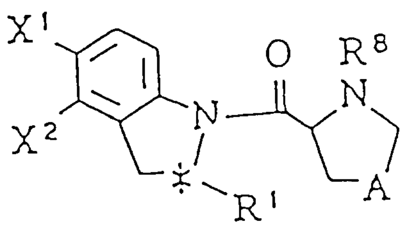

(IIa)

von der Verbindung (3) und Hydrolysierung der optisch aktiven Verbindung (IIa), unter Erhalt einer Verbindung, dargestellt durch die allgemeine Formel (IIb):

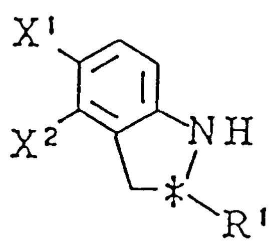

(IIb)

worin $R^1$, $X^1$ und $X^2$ die gleichen Bedeutungen wie oben haben und worin * die gleiche Bedeutung wie in Anspruch 1 hat,

c) Reaktion der Verbindung (IIb) mit Dialkylethoxymethylenmalonat, unter Erhalt einer Verbindung, dargestellt durch die allgemeine Formel (4):

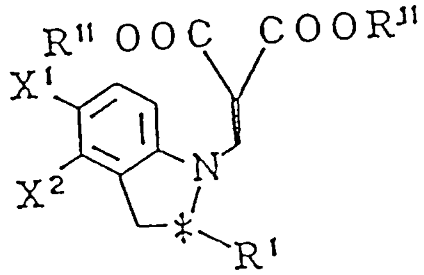

(4)

worin $R^1$, $X^1$ und $X^2$ die gleichen Bedeutungen wie oben haben, worin $R^{11}$ eine $C_1$-$C_6$-Alkyl-Gruppe ist und * die gleiche Bedeutung wie in Anspruch 1 hat,

d) Cyclisierung und Hydrolysierung der Verbindung (4), unter Erhalt einer Verbindung, dargestellt durch die allgemeine Formel (IV):

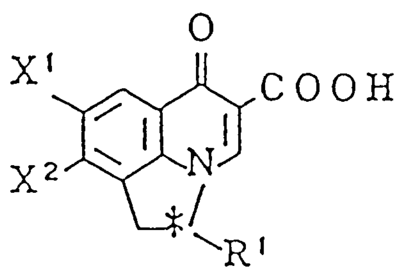

(IV)

worin $R^1$, $X^1$ und $X^2$ die gleichen Bedeutungen wie oben haben und worin * die gleiche Bedeutung wie in Anspruch 1 hat,

e) Reaktion der Verbindung (IV) mit der Verbindung, dargestellt durch die allgemeine Formel (5):

$$R^2H \qquad (5)$$

worin $R^2$ eine Gruppe ist:

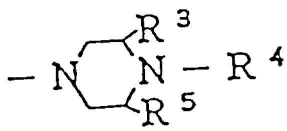

worin $R^3$, $R^4$ und $R^5$ gleich oder verschieden sind, und ein Wasserstoffatom oder eine $C_1$-$C_6$-Alkyl-Gruppe sind, vorausgesetzt, daß zumindest eine der Gruppen von $R^3$, $R^4$ und $R^5$ die $C_1$-$C_6$-Alkyl-Gruppe ist, unter Erhalt einer Verbindung, dargestellt durch eine allgemeine Formel (I):

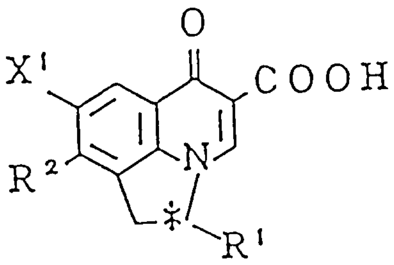

(I)

worin $R^1$, $R^2$ und $X^1$ die gleichen Bedeutungen wie oben haben und * die gleiche Bedeutung wie in Anspruch 1 hat,

f) Bildung einer Chelat-Verbindung, dargestellt durch die allgemeine Formel (6):

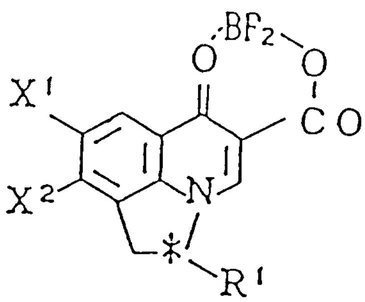

(6)

worin $R^1$, $X^1$, $X^2$ die gleichen Bedeutungen wie oben haben und * die gleiche Bedeutung wie in Anspruch 1 hat, von der Verbindung (4) oder (IV) und anschließende Reaktion der Chelat-Verbindung (6) mit der Verbindung (5), unter Erhalt der Verbindung (I);

g) Reaktion der Verbindung (1), die ein Racemat ist, mit einer optisch aktiven Verbindung, zur Bildung eines Salzes der Verbindung (1), und anschließende Durchführung einer fraktionierten Kristallisierung und eines Entsalzungsschrittes, unter Erhalt der optisch aktiven Verbindung (IIb).

**28.** Antimikrobielles Mittel, umfassend als einen aktiven Bestandteil ein Pyrrolochinolin-Derivat oder Salz davon nach Anspruch 1.

**29.** Antimikrobielles Mittel, umfassend als einen aktiven Bestandteil ein pyrrolochinolin-Derivat oder Salz davon nach Anspruch 18.

**30.** Antimikrobielles Mittel, umfassend als einen aktiven Bestandteil ein Pyrrolochinolin-Derivat oder Salz davon nach Anspruch 2.

**31.** Antimikrobielles Mittel, umfassend als einen aktiven Bestandteil ein Pyrrolochinolin-Derivat oder Salz davon nach Anspruch 3.

**32.** Antimikrobielles Mittel, umfassend als einen aktiven Bestandteil ein Pyrrolochinolin-Derivat oder Salz davon nach Anspruch 25.

**33.** Antimikrobielles Mittel, umfassend als einen aktiven Bestandteil ein Pyrrolochinolin-Derivat oder Salz davon nach Anspruch 26.

**34.** Verwendung eines Pyrrolochinolin-Derivates oder eines Salzes davon nach den Ansprüchen 1, 18 oder 21, zur Herstellung einer antimikrobiellen Zusammensetzung für Menschen und Tiere.

## Revendications

**1.** Dérivé de pyrroloquinoléine de la formule générale (I) :

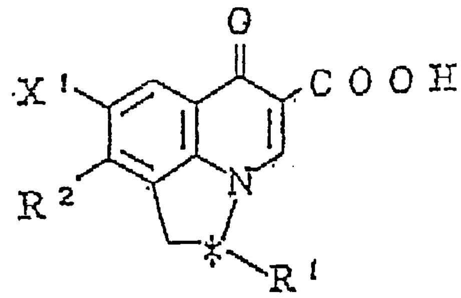

(I)

dans laquelle $R^1$ est un groupe alkyle en $C_1$-$C_6$; $R^2$ est un groupe

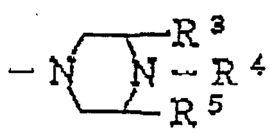

dans lequel $R^3$, $R^4$ et $R^5$ sont identiques ou différents, un atome d'hydrogène ou un groupe alkyle en $C_1$-$C_6$, à condition qu'au moins l'un des groupes $R^3$, $R^4$ et $R^5$ soit le groupe alkyle en $C_1$-$C_6$, $X^1$ est un atome d'halogène et * représente un atome asymétrique, qui a une activité optique grâce à l'atome de carbone asymétrique à la position 2 où l'atome de carbone asymétrique à la position 2 du cycle pyrroloquinoléine a la même configuration que l'atome de carbone asymétrique à la position 2 de l'un des deux diastéréoisomères du (4,5-difluoro-2-méthyl-1-[(S)-N-p-toluènesulfonylprolyl]-2,3-dihydroindole), qui présentent un $[a]_D^{20}$ = -10,38° (c = 1,06, chloroforme)), ou son sel.

**2.** Dérivé de pyrroloquinoléine ou son sel selon la revendication 1, dans lequel $R^2$ est un groupe :

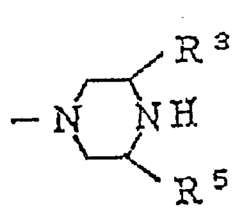

dans lequel $R^3$ et $R^5$ sont identiques ou différents, un atome d'hydrogène ou un groupe alkyle en $C_1$-$C_6$, à condition qu'au moins l'un des groupes $R^3$ et $R^5$ soit le groupe alkyle en $C_1$-$C_6$.

**3.** Dérivé de pyrroloquinoléine ou son sel selon la revendication 1, dans lequel $R^2$ est un groupe :

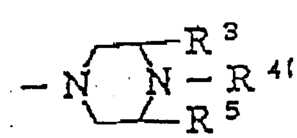

dans lequel $R^3$ et $R^5$ sont identiques ou différents, un atome d'hydrogène ou un groupe alkyle en $C_1$-$C_6$, et $R^{4'}$ est un groupe alkyle en $C_1$-$C_6$.

4. Dérivé de pyrroloquinoléine ou son sel selon la revendication 2, dans lequel le groupe alkyle est un groupe méthyle ou un groupe éthyle et $X^1$ est un atome de fluor ou un atome de chlore.

5. Dérivé de pyrroloquinoléine ou son sel selon la revendication 4, dans lequel $X^1$ est un atome de fluor.

6. Dérivé de pyrroloquinoléine ou son sel selon la revendication 4, dans lequel le groupe alkyle est un groupe méthyle, et $X^1$ est un atome de chlore.

7. Dérivé de pyrroloquinoléine ou son sel selon la revendication 5, dans lequel le groupe alkyle est un groupe méthyle.

8. Sel d'un dérivé de pyrroloquinoléine selon la revendication 7.

9. Dérivé de pyrroloquinoléine, qui n'est pas un sel, selon la revendication 7.

10. Sel d'un dérivé de pyrroloquinoléine selon la revendication 6.

11. Dérivé de pyrroloquinoléine, qui n'est pas un sel, selon la revendication 6.

12. Sel d'un dérivé de pyrroloquinoléine selon la revendication 3.

13. Dérivé de pyrroloquinoléine, qui n'est pas un sel, selon la revendication 3.

14. Dérivé de pyrroloquinoléine ou son sel selon la revendication 12, dans lequel le groupe alkyle est un groupe méthyle, et $X^1$ est un atome de fluor.

15. Dérivé de pyrroloquinoléine ou son sel selon la revendication 13, dans lequel le groupe alkyle est un groupe méthyle, et $X^1$ est un atome de fluor.

16. Dérivé de pyrroloquinoléine ou son sel selon la revendication 14, dans lequel l'un de $R^3$ et $R^5$ est un atome d'hydrogène.

17. Dérivé de pyrroloquinoléine ou son sel selon la revendication 14, dans lequel $R^3$ et $R^5$ sont des groupes alkyle en $C_1$-$C_6$.

18. Dérivé de pyrroloquinoléine de la formule générale III :

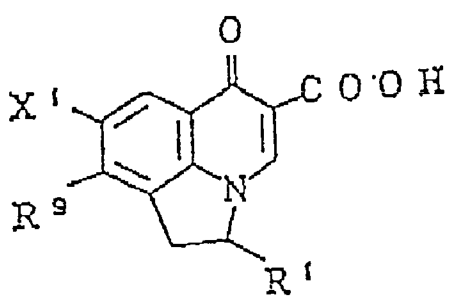

(III)

dans laquelle $R^1$ est un groupe alkyle en $C_1$-$C_6$ ; $R^9$ est un groupe :

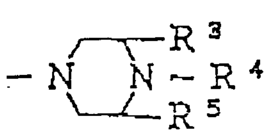

dans lequel $R^3$ est un groupe alkyle en $C_1$-$C_6$, et $R^4$ et $R^5$ sont des atomes d'hydrogène, $X^1$ est un atome d'halogène ou un de ses sels qui est une modification racémique.

**19.** Dérivé de pyrroloquinoléine ou son sel selon la revendication 18, dans lequel $X^1$ est un atome de fluor, $R^1$ est un groupe méthyle, et $R^9$ est un groupe:

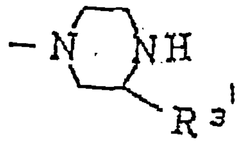

dans lequel $R^{3'}$ est un groupe méthyle ou un groupe éthyle.

**20.** Composé optiquement actif de la formule générale (II) :

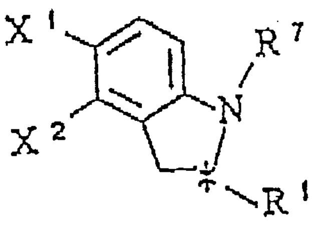

(II)

dans laquelle $R^1$ est un groupe alkyle en $C_1$-$C_6$, $R^7$ est un atome d'hydrogène ou un groupe :

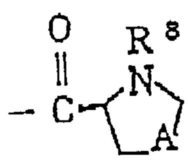

dans lequel $R^8$ est un groupe protecteur choisi parmi les groupes phénylsulfonyle substitués par un groupe alkyle en $C_1$-$C_6$, et les groupes phénylcarbonyle substitués par un groupe alkyle en $C_1$-$C_6$; A est un groupe alkylène en $C_1$-$C_4$, $X^1$ et $X^2$ sont des atomes d'halogène, et * représente un atome asymétrique qui a une activité optique due à l'atome de carbone asymétrique à la position 2, qui a la même configuration que l'atome de carbone asymétrique à la position de 2 de l'un des deux diastéréoisomères du 4,5-difluoro-2-méthyl-1-[(S)-N-p-toluènesulfonyl-propyl]-2,3-dihydroindole qui présentent un $[\alpha]_D^{20}$ = -10,38° (c = 1,06, chloroforme).

**21.** Dérivé de pyrroloquinoléine de la formule générale IV :

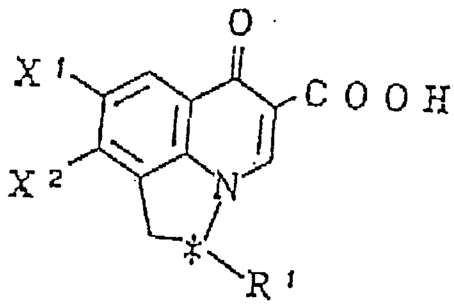

(IV)

dans laquelle $R^1$ est un groupe alkyle en $C_1$-$C_6$, $X^1$ et $X^2$ sont des atomes d'halogène, et * représente un atome de carbone asymétrique, dans lequel l'atome de carbone asymétrique à la position 2 du cycle pyrroloquinoléine a la même configuration que l'atome de carbone symétrique à la position 2 de l'un des deux diastéréoisomères du 4,5-difluoro-2-méthyl-1-[(S)-N-p-toluènesulfonylprolyl]-2,3-dihydroindole qui présentent un $[\alpha]_D^{20}$ = -10,38° (c = 1,06, chloroforme), ou un de ses sels.

**22.** Chlorhydrate de l'acide (+)-8-fluoro-2-méthyl-9-(3-méthyl-1-pipérazinyl)-6-oxo-1,2-dihydro-6H-pyrrolo[3,2,1-ij]quinoléine-5-carboxylique ($[\alpha]^{20}$ = + 27,52° (c = 0,545, 1N NaOH)),
chlorhydrate de l'acide (±)-8-fluoro-2-méthyl-9-(3-méthyl-1-pipérazinyl)-6-oxo-1,2-dihydro-6H-pyrrolo[3,2,1-ij]quinoléine-5-carboxylique,
chlorhydrate de l'acide (+)-8-fluoro-2-méthyl-9-(3-méthyl-1-pipérazinyl)-6-oxo-1,2-dihydro-6H-pyrrolo[3,2,1-ij]quinoléine-5-carboxylique ($[\alpha]^{20}$ = + 15,5°(c = 0,50, 1N NaOH)),
chlorhydrate de l'acide (+)-8-fluoro-2-méthyl-9-(3,5-diméthyl-1-pipérazinyl)-6-oxo-1,2-dihydro-6H-pyrrolo[3,2, 1-ij]quinoléine-5-carboxylique ($[\alpha]^{20}$ = + 10,59° (c = 0,85, 1N NaOH)),
chlorhydrate de l'acide (-)-8-fluoro-2-méthyl-9-(3-S-méthyl-1-pipérazinyl)-6-oxo-1,2-dihydro-6H-pyrrolo[3,2,1-ij]quinoléine-5-carboxylique ($[\alpha]^{20}$ = -1,67° (c = 0,60, 1N NaOH)), ou
chlorhydrate de l'acide (+)-8-fluoro-2-méthyl-9-(3-R-méthyl-1-pipérazinyl)-6-oxo-1,2-dihydro-6H-pyrrolo[3,2,1-ij]quinoléine-5-carboxylique ($[\alpha]^{20}$ = + 35,29° (c = 0,68, 1N NaOH)) selon la revendication 4.

**23.** Acide (+)-8-fluoro-2-méthyl-9-(4-méthyl-1-pipérazinyl)-6-oxo-1,2-dihydro-6H-pyrrolo[3,2,1-ij]quinoléine-5-carboxylique ($[\alpha]^{20}$ = + 9,24° (c = 0,866, DMSO)),
chlorhydrate de l'acide (+)-8-fluoro-2-méthyl-9-(4-méthyl-1-pipérazinyl)-6-oxo-1,2-dihydro-6H-pyrrolo[3,2,1-ij]quinoléine-5-carboxylique, ($[\alpha]^{20}$ = + 10,70° (c = 0,873, 1N NaOH)),
acide (+)-8-fluoro-2-méthyl-9-(3,4-diméthyl-1-pipérazinyl)-6-oxo-1,2-dihydro-6H-pyrrolo[3,2,1-ij]quinoléine-5-carboxylique ($[\alpha]^{20}$ = + 10,53° (c = 0,57, 1N NaOH)), chlorhydrate de l'acide (+)-8-fluoro-2-méthyl-9-(3,4-diméthyl-1-pipérazinyl)-6-oxo-1,2-dihydro-6H-pyrrolo-[3,2,1-ij]quinoléine-5-carboxylique ($[\alpha]^{20}$ = + 12,13° (c = 0,58, 1N NaOH)),
acide (+)-8-fluoro-2-méthyl-9-(3,4,5-triméthyl-1-pipérazinyl)-6-oxo-1,2-dihydro-6H-pyrrolo[3,2,1-ij]quinoléine-5-carboxylique ($[\alpha]^{20}$ = + 16,57° (c = 0,905, 1N NaOH)), chlorhydrate de l'acide (+)-8-fluoro-2-méthyl-9-(3,4,5-triméthyl-1-pipérazinyl)-6-oxo-1,2-dihydro-6H-pyrrolo-[3,2,1-ij]quinoléine-5-carboxylique ($[\alpha]^{20}$ = + 18,99° (c = 0,913, 1N NaOH)),
acide (+)-8-fluoro-2-méthyl-9-((3S)-3,4-diméthyl-1-pipérazinyl)-6-oxo-1,2-dihydro-6H-pyrrolo[3,2,1-ij]quinoléine-5-carboxylique ($[\alpha]_D^{20}$ = + 2,86° (c = 0,35, 1N NaOH)), ou
chlorhydrate de l'acide (+)-8-fluoro-2-méthyl-9-((3S)-3,4-diméthyl-1-pipérazinyl)-6-oxo-1,2-dihydro-6H-pyrrolo[3,2,1-ij]quinoléine-5-carboxylique ($[\alpha]^{20}$ = + 3,30° (c = 0,36, 1N NaOH)) selon la revendication 5.

**24.** Acide (+)-8,9-difluoro-2-méthyl-6-oxo-1,2-dihydro-6H-pyrrolo[3,2,1-ij]quinoléine-5-carboxylique ($[\alpha]^{20}$ = + 26,32° (c = 0,57, DMSO)) selon la revendication 21.

**25.** Chlorhydrate de l'acide (+)-8-fluoro-2-méthyl-9-(3-méthyl-1-pipérazinyl)-6-oxo-1,2-dihydro-6H-pyrrolo[3,2,1-ij]quinoléine-5-carboxylique ($[\alpha]^{20}$ = + 27,52° (c = 0,545, 1N NaOH)),
chlorhydrate de l'acide (-)-8-fluoro-2-méthyl-9-(3-S-méthyl-1-pipérazinyl)-6-oxo-1,2-dihydro-6H-pyrrolo[3,2, 1-ij]quinoléine-5-carboxylique, ($[\alpha]^{20}$ = + 1,67° (c = 0,60, 1N NaOH)) ou
chlorhydrate de l'acide (+)-8-fluoro-2-méthyl-9-(3-R-méthyl-1-pipérazinyl)-6-oxo-1,2-dihydro-6H-pyrrolo[3,2, 1-ij]quinoléine-5-carboxylique ($[\alpha]^{20}$ = + 35,29° (c = 0,68, 1N NaOH)), selon la revendication 22.

**26.** Acide (+)-8-fluoro-2-méthyl-9-(3,4-diméthyl-1-pipérazinyl)-6-oxo-1,2-dihydro-6H-pyrrolo[3,2, 1-ij]quinoléine-5-carboxylique ([α]$^{20}$ = + 10,53° (c = 0,57, 1N NaOH)),
chlorhydrate de l'acide (+)-8-fluoro-2-méthyl-9-(3,4-diméthyl-1-pipérazinyl)-6-oxo-1,2-dihydro-6H-pyrrolo[3,2, 1-ij]quinoléine-5-carboxylique ([α]$^{20}$ = + 12,13° (c = 0,58, 1N NaOH)),
acide (+)-8-fluoro-2-méthyl-9-((3S)-3,4-diméthyl-1-pipérazinyl)-6-oxo-1,2-dihydro-6H-pyrrolo[3,2,1-ij]quinoleine-5-carboxylique ([α]$_D^{20}$ = + 2,86° (c = 0,35, 1N NaOH)), ou
chlorhydrate de l'acide (+)-8-fluoro-2-méthyl-9-((3S)-3,4-diméthyl-1-pipérazinyl)-6-oxo-1,2-dihydro-6H-pyrrolo[3,2,1-ij]quinoléine-5-carboxylique ([α]$_D^{20}$ = + 3,30° (c = 0,36, 1N NaOH)), selon la revendication 23.

**27.** Procédé pour préparer un dérivé de pyrroloquinoléine selon les revendications 1 ou 21, qui consiste à :

a) faire réagir un composé représenté par la formule générale (1) :

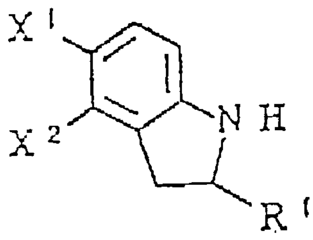

(1)

dans laquelle $R^1$ est un groupe alkyle en $C_1$-$C_6$, $X^1$ et $X^2$ sont des atomes d'halogène, avec un composé représenté par la formule générale (2) :

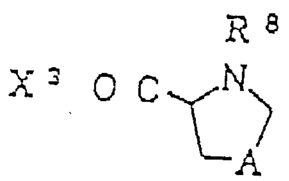

(2)

dans laquelle $R^8$ est un groupe protecteur, A est un groupe alkylène en $C_1$-$C_4$, $X^3$ est un groupe hydroxy, un atome d'halogène ou un reste d'un dérivé réactif d'un groupe carboxyle, pour obtenir un composé représenté par la formule générale (3) :

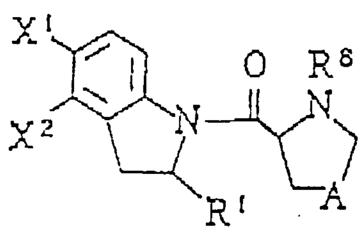

(3)

dans laquelle $R^1$, $R^8$, $X^1$, $X^2$ et A ont les mêmes significations que ci-dessus,

b) séparer un composé optiquement actif représenté par la formule générale (IIa) :

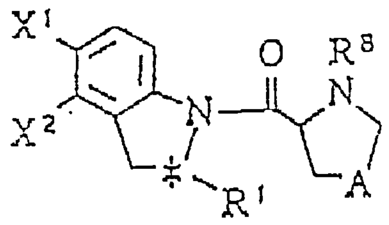

(IIa)

dudit composé (3), et hydrolyser le composé optiquement actif (IIa) pour obtenir un composé représenté par la formule générale (IIb) :

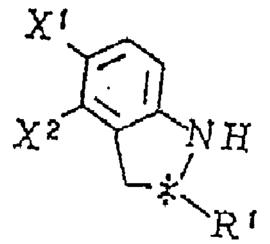

(IIb)

dans laquelle $R^1$, $X^1$ et $X^2$ ont les mêmes significations que ci-dessus, et * a la même signification que celle définie en revendication 1,

c) faire réagir ledit composé (IIb) avec du dialkyléthoxyméthylènemalonate pour obtenir un composé représenté par la formule générale (4) :

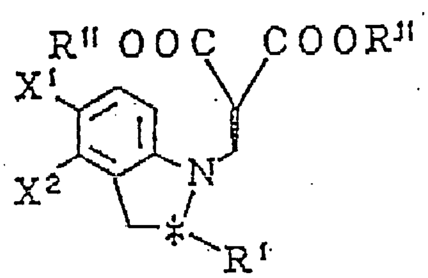

(4)

dans laquelle $R^1$, $X^1$ et $X^2$ ont les mêmes significations que ci-dessus, $R^{11}$ est un groupe alkyle en $C_1$-$C_6$, et * a la même signification que celle définie en revendication 1,

d) cycliser et hydrolyser ledit composé (4) pour obtenir un composé représenté par la formule générale (IV) :

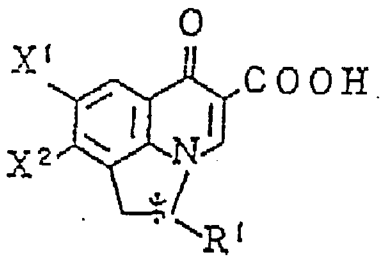

(IV)

dans laquelle $R^1$, $X^1$ et $X^2$ ont les mêmes significations que ci-dessus, et * a la même signification que celle définie en revendication 1.

e) faire réagir ledit composé (IV) avec le composé représenté par la formule générale (5) :

$R^2H$ (5)

dans laquelle $R^2$ est un groupe :

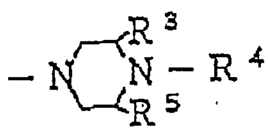

dans lequel $R^3$, $R^4$ et $R^5$ sont identiques ou différents, un atome d'hydrogène ou un groupe alkyle en $C_1$-$C_6$, à condition qu'au moins l'un des groupes $R^3$, $R^4$ et $R^5$ soit le groupe alkyle en $C_1$-$C_6$, afin d'obtenir un composé représenté par la formule générale (I) :

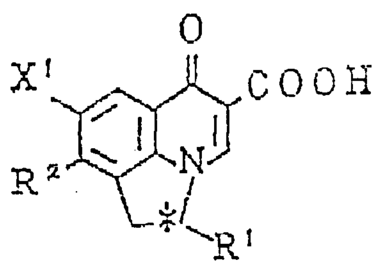

(I)

dans laquelle $R^1$, $R^2$ et $X^1$ ont les mêmes significations que ci-dessus, et * a la même signification que celle définie en revendication 1,

f) former un chélate représenté par la formule générale (6) :

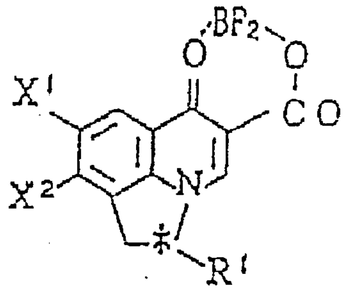

(6)

dans laquelle $R^1$, $X^1$, $X^2$ ont les mêmes significations que ci-dessus, et * a la même signification que celle définie en revendication 1, à partir dudit composé (4) ou (IV), et ensuite faire réagir le chélate (6) avec ledit composé (5) pour obtenir ledit composé (I);

g) faire réagir ledit composé (1), qui est un racémate, avec un composé optiquement actif pour former un sel du composé (1), et ensuite accomplir une cristallisation fractionnée et éliminer les sels pour obtenir ledit composé optiquement actif (IIb).

**28.** Agent antimicrobien contenant, comme ingrédient actif, un dérivé de pyrroloquinoléine ou son sel, tel que défini en revendication 1.

**29.** Agent antimicrobien contenant, comme ingrédient actif, un dérivé de pyrroloquinoléine ou son sel, tel que défini en revendication 18.

**30.** Agent antimicrobien contenant, comme ingrédient actif, un dérivé de pyrroloquinoléine ou son sel, tel que défini en revendication 2.

**31.** Agent antimicrobien contenant, comme ingrédient actif, un dérivé de pyrroloquinoléine ou son sel, tel que défini en revendication 3.

**32.** Agent antimicrobien contenant, comme ingrédient actif, un dérivé de pyrroloquinoléine ou son sel, tel que défini en revendication 25.

**33.** Agent antimicrobien contenant, comme ingrédient actif, un dérivé de pyrroloquinoléine ou son sel, tel que défini en revendication 26.

**34.** Utilisation d'un dérivé de pyrroloquinoléine ou son sel, tel que défini en revendications 1, 18 ou 21 pour préparer une composition antimicrobienne pour les êtres humains et les animaux.

Fig. 1

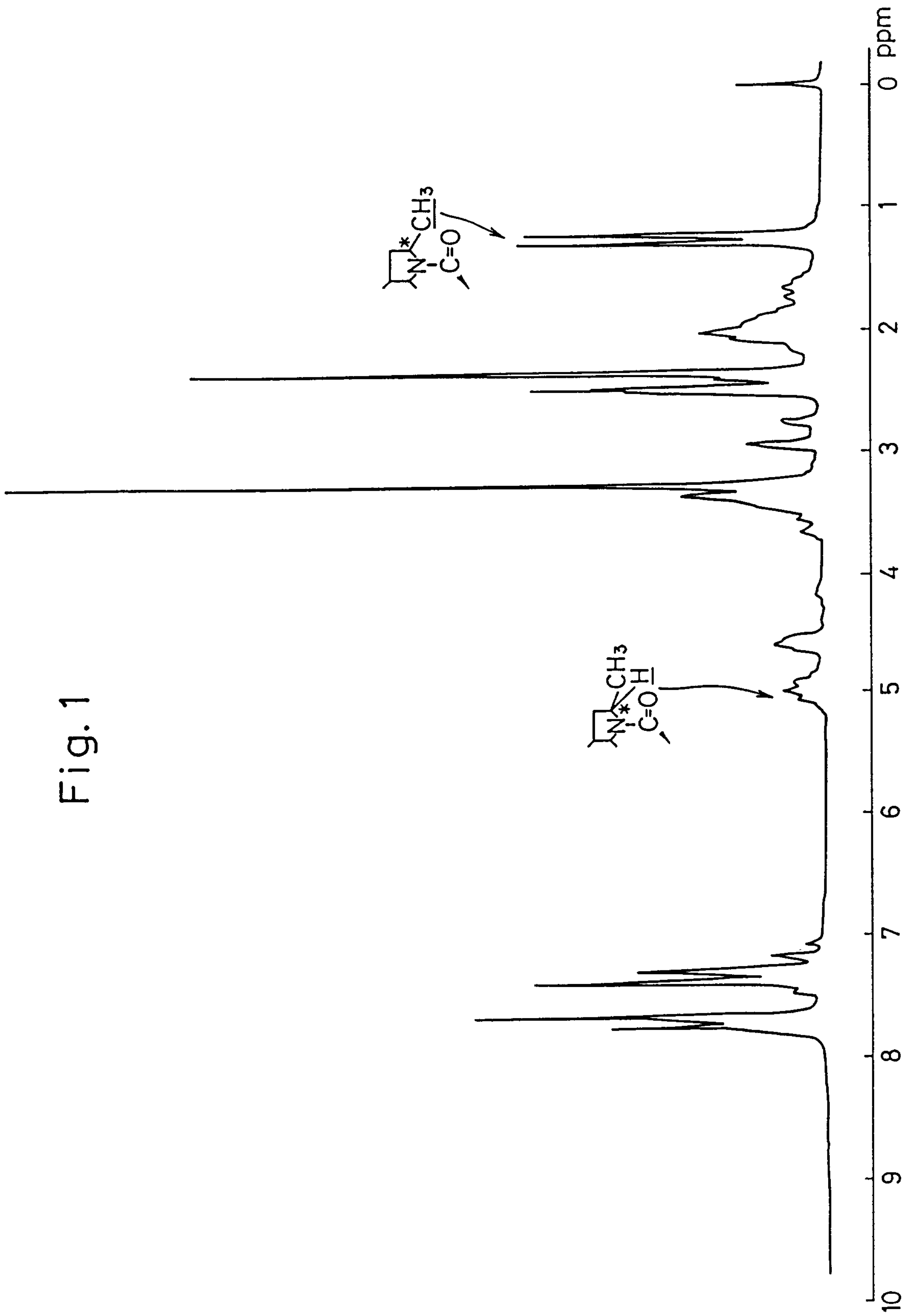

Fig. 2